(19) Europäisches Patentamt
European Patent Office
Office européen des brevets

(11) Veröffentlichungsnummer: **0 176 034**
**B1**

(12) **EUROPÄISCHE PATENTSCHRIFT**

(45) Veröffentlichungstag der Patentschrift: **30.08.89**

(21) Anmeldenummer: **85111837.2**

(22) Anmeldetag: **19.09.85**

(51) Int. Cl.[4]: **C 07 C 63/66**, C 07 C 65/28, C 07 C 69/76, C 07 C 69/157, C 07 C121/64, C 07 C121/75, C 07 C 33/38, C 07 C 47/548, C 07 C 13/48, C 07 C 25/24, C 07 C 43/215

(54) **Diarylacetylene, ihre Herstellung und Verwendung.**

(30) Priorität: **22.09.84 DE 3434946**

(43) Veröffentlichungstag der Anmeldung: **02.04.86 Patentblatt 86/14**

(45) Bekanntmachung des Hinweises auf die Patenterteilung: **30.08.89 Patentblatt 89/35**

(84) Benannte Vertragsstaaten: **AT BE CH DE FR GB IT LI NL SE**

(56) Entgegenhaltungen:
**EP-A- 0 002 742**
**EP-A- 0 084 667**

(73) Patentinhaber: **BASF Aktiengesellschaft**
**Carl-Bosch-Strasse 38**
**D-6700 Ludwigshafen (DE)**

(72) Erfinder: **Wuest, Hans-Heiner, Dr.**
**Unteres Bieth 10**
**D-6901 Dossenheim (DE)**
Erfinder: **Frickel, Fritz-Frieder, Dr.**
**Silvanerweg 7**
**D-6705 Deidesheim (DE)**
Erfinder: **Nuerrenbach, Axel, Dr.**
**Köningsberger Strasse 7**
**D-6718 Gruenstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 176 034 B1

Jouve, 18, rue St-Denis, 75001 Paris, France

## Beschreibung

Die Erfindung betrifft neue Diarylacetylene, Verfahren zu deren Herstellung sowie deren Verwendung bei der Bekämpfung von Krankheiten.

Aus EP-A-84 667 und EP-A-2 742 ist bekannt, daß Stilbenderivate pharmakologische Wirkungen bei der topischen und systemischen Therapie von Neoplasien, Akne, Psoriasis und anderen dermatologischen Affektionen aufweisen. Es wurde nun gefunden, daß Diarylacetylene ein besseres Wirkungsspektrum besitzen.

Erfindungsgegenstand sind Diarylacetylene der Formel I

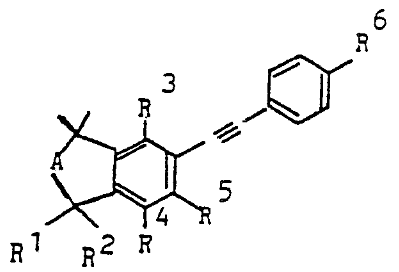

(I)

in der

$R^1$ und $R^2$ Wasserstoffatome oder Methylgruppen

$R^3$ ein Wasserstoffatom oder eine Methyl-, Hydroxy- oder $C_{1-6}$-Alkoxygruppe

$R^4$ ein Wasserstoffatom oder eine Methyl- oder Methoxygruppe

$R^5$ ein Wasserstoff- oder Halogenatom oder eine Methoxy- der $C_{1-4}$-Alkylgruppe

A einen gegebenenfalls mit $C_{1-4}$-Alkylgruppen substituierten Methylen- oder Ethylenrest oder die Gruppen $-CH=CH-$, $-CHOH-CH_2-$ oder $-CO-CH_2-$ und

$R^6$ ein Wasserstoffatom, eine Methyl- oder Nitrilgruppe, den Tetrazolyl- oder 2-Oxazolinylrest, eine $C_{2-10}$-Ketalgruppe oder die Reste $-CHR^7-OR^8$, $-CHR^8-NR^9R^{10}$, $-COR^{11}$, $-CR^{12}=CH-COOR^{13}$ oder $-CR^{12}=CH-CO-NR^{14}R^{15}$ bedeuten, worin

$R^7$ ein Wasserstoffatom oder eine $C_{1-4}$-Alkylgruppe

$R^8$ ein Wasserstoffatom, eine $C_{1-4}$-Alkyl-, $C_{1-20}$-Alkanoyl-, eine gegebenenfalls substituierte Benzoylgruppe oder die Reste $-P(O)(OR^{13})_2$ oder $-P(O)(NR^{14}R^{15})_2$ (mit $R^{13}$ in der Bedeutung eines Wasserstoffatoms, einer gegebenenfalls durch eine Hydroxygruppe substituierten $C_{1-8}$-Alkylgruppe, einer gegebenenfalls substituierten Aryl- oder gegebenenfalls im Arylteil substituierten Aralkylgruppe, und mit $R^{14}$ und $R^{15}$ in der Bedeutung von Wasserstoffatomen, gegebenenfalls durch eine Hydroxygruppe substituierten $C_{1-6}$-Alkylgruppen, gegebenenfalls substituierten Aralkyl- oder Arylgruppen, oder $R^{14}$ und $R^{15}$ zusammen mit dem Stickstoffatom, an das sie gebunden sind, in der Bedeutung eines heterocyclischen Restes

$R^9$ und $R^{10}$ Wasserstoffatome, $C_{1-4}$-Alkyl-, $C_{1-6}$-Alkanoyl- oder gegebenenfalls substituierten Benzolgruppen oder zusammen mit dem Stickstoffatom, an das sie gebunden sind, einen heterocyclischen Rest

$R^{11}$ ein Wasserstoff- oder Halogenatom, einer $C_{1-4}$-Alkylgruppe oder die Reste $-NR^{14}R^{15}$ oder $-OR^{13}$ (mit $R^{13}$, $R^{14}$ und $R^{15}$ in der oben angegebenen Bedeutung) und

$R^{12}$ ein Wasserstoffatom oder eine Methylgruppe darstellen,

sowie gegebenenfalls deren physiologisch verträgliche Salze.

Von den Verbindungen der Formel I sind diejenigen bevorzugt, in denen A ein durch die Methylgruppe substituierten Methylen- oder Ethylenrest bedeutet. Stellen $R^9$ und $R^{10}$ zusammen mit dem Stickstoffatom, an das sie gebunden sind, einen heterocyclischen Rest dar, so ist dieser vorzugsweise die Pyrrolidino-, Piperidino- oder Morpholinogruppe. Sind $R^5$ und $R^{11}$ Halogenatome, so ist bei $R^5$ Fluor und bei $R^{11}$ Chlor und Brom besonders zu nennen. Bevorzugte Beispiele von Substituenten der Benzoylgruppe (vgl. $R^8$, $R^9$, $R^{10}$) sind die Methoxy-, Nitro- oder Methylgruppen oder Halogenatome, insbesondere Chlor oder Brom. Bevorzugt als Arylrest ($R^{13}$, $R^{14}$, $R^{15}$) ist die Phenylgruppe, die gegebenenfalls durch eine Methyl-, Methyl- oder Nitrogruppe substituiert sein kann. Als Aralkylgruppe ($R^{13}$, $R^{14}$, $R^{15}$) ist die Benzylgruppe bevorzugt, die im Arylteil insbesondere durch Methyl, Methoxy oder Halogen substituiert sein kann. Als Beispiele für heterocyclische Reste $-NR^9R^{10}$ und $-NR^{14}R^{15}$ kommen insbesondere Pyrrolidino, Piperidino oder Morpholino in Betracht.

Typische Beispiele für erfindungsgemäße Verbindungen sind :

4-[(5,6,7,8-Tetrahydro-5,5,8,8-tetramethyl-2-naphthyl) ethinyl] benzoesäure

4-[(5,6,7,8-Tetrahydro-3,5,5,8,8-pentamethyl-2-naphthyl) ethinyl] benzoesäure

4-[(3-Ethyl-5,6,7,8-tetrahydro-5,5,8,8-tetramethyl-2-naphthyl) ethinyl] benzoesäure

4-[(3-Fluor-5,6,7,8-tetrahydro-5,5,8,8-tetramethyl-2-naphthyl) ethinyl] benzoesäure

4-[(5,6,7,8-Tetrahydro-3-methoxy-5,5,8,8-tetramethyl-2-naphthyl) ethinyl] benzoesäure
4-[(5,6,7,8-Tetrahydro-1-hydroxy-5,5,3,8-tetramethyl-2-naphthyl) ethinyl] benzoesäure
4-[(5,6,7,8-Tetrahydro-1-methoxy-5,5,8,8-tetramethyl-2-naphthyl) ethinyl] benzoesäure
4-[(5,6,7,8-Tetrahydro-1,3-dimethoxy-5,5,8,8-tetramethyl-2-naphthyl) ethinyl] benzoesäure
4-[(5,6,7,8-Tetrahydro-1,4-dimethoxy-5,5,8,8-tetramethyl-2-naphthyl) ethinyl] benzoesäure
4-[(5,6,7,8-Tetrahydro-1-methoxy-4,5,5,8,8-pentamethyl-2-naphthyl) ethinyl] benzoesäure
4-[(5,6,7,8-Tetrahydro-1-methoxy-3,5,5,8,8-pentamethyl-2-naphthyl) ethinyl] benzoesäure
4-[(5,6,7,8-Tetrahydro-1,4-dimethoxy-3,5,5,8,8-pentamethyl-2-naphthyl) ethinyl] benzoesäure
4-[(5,6,7,8-Tetrahydro-5,5,8,8-tetramethyl-7-oxo-2-naphthyl) ethinyl] benzoesäure
4-[(5,6,7,8-Tetrahydro-7-hydroxy-5,5,8,8-tetramethyl-2-naphthyl) ethinyl] benzoesäure
4-[(5,6,7,8-Tetrahydro-3,8,8-trimethyl-2-naphthyl) ethinyl] benzoesäure
4-[(2,3-Dihydro-1,1,3,3-tetramethyl-5(1H)-indenyl) ethinyl] benzoesäure
4-[(2,3-Dihydro-1,1,2,3,3-pentamethyl-5(1H)-indenyl) ethinyl] benzoesäure
4-[(2,3-Dihydro-1,1,2,3,3,6-hexamethyl-5(1H)-indenyl) ethinyl] benzoesäure
4-[(5,6,7,8-Tetrahydro-5,5,6,8,8-pentamethyl-2-naphthyl) ethinyl] benzoesäure
4-[(5,8-Dihydro-5,5,8,8-tetramethyl-2-naphthyl) ethinyl] benzoesäure
4-[(1-Hexyl-5,6,7,8-tetrahydro-3,5,5,8,8-pentamethyl-2-naphthyl) ethinyl] benzoesäure

Typisch sind weiter Verbindungen, die anstelle der Carboxylgruppe folgende Reste enthalten :

Methoxycarbonyl-, Ethoxycarbonyl-, Propyloxycarbonyl, Butoxycarbonyl-, Benzyloxycarbonyl-, Chlorcarbonyl-, Cyano-, Formyl-, Hydroxymethyl-, Methyl-, Acetyl-, Methoxymethyl-, Ethoxymethyl-, Benzyloxymethyl-, Formyloxymethyl-, Acetoxymethyl-, Propionyloxymethyl-, Hexadecanoyloxymethyl-, Benzyloxymethyl-, (3,4-Dimethoxy) benzyloxymethyl-, Dihydroxyphosphoryloxymethyl-, Dimethoxyphosphoryloxymethyl-, Bis(dimethylamido) phosphoryloxymethyl-, Aminomethyl-, Methylaminomethyl-, Ethylaminomethyl-, Propylaminomethyl-, Butylaminomethyl-, Acetylaminomethyl-, Formylaminomethyl-, Benzoylaminomethyl-, (4-Methoxy) benzoylaminomethyl-, Dimethylaminomethyl-, Morpholinomethyl-, Pyrrolidinomethyl-, Piperidinomethyl-, Oxazolin-2-yl, Tetrazol-5-yl-, 1,3-Dioxolan-2-yl-, Dimethoxymethyl-, (E)-2-Carbethoxyethenyl-, (E)-2-Carboxyethenyl-, Wasserstoff, Carbamoyl-, Methylcarbamoyl-, Dimethylcarbamoyl-, Morpholinocarbonyl-, Phenylcarbamoyl-.

Die erfindungsgemäßen Verbindungen kann man herstellen, indem man

a) falls $R^6$ eine Cyanogruppe bedeutet — Stilbene der Formel II

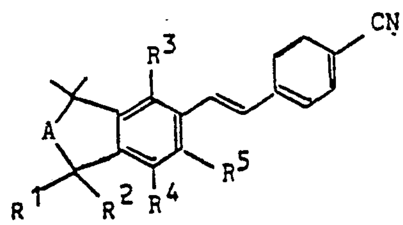

(II)

in der $R^1$-$R^5$ und A die oben angegebene Bedeutung haben, halogeniert und anschließend 2 Mole Halogenwasserstoff abspaltet, oder

b) falls $R^6$ ein Wasserstoffatom, eine Carboxyl- Nitril- oder Formylgruppe bedeutet — ein α-Chlorbenzylphosphonat der Formel III

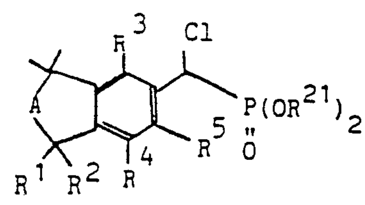

(III)

in der $R^1$-$R^5$ und A die oben angegebene Bedeutung haben, und $R^{21}$ eine $C_{1-3}$-Alkylgruppe bedeutet, mit Aldehyden der Formel IV

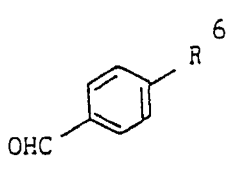

(IV)

umsetzt oder

c) falls $R^6$ eine Methyl- oder Nitrilgruppe oder einen 1,3-Dioxolan-2-yl-rest bedeutet — ein α-Chlorbenzylphosphonat der Formel V,

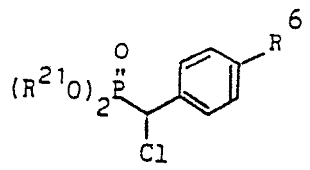

(V)

worin $R^{21}$ die oben angegebene Bedeutung hat, mit Aldehyden der Formel VI

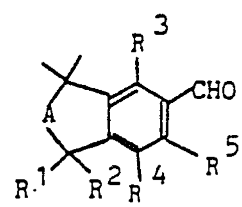

(VI)

worin $R^1$-$R^5$ und A die oben angegebene Bedeutung haben, umsetzt oder

d) falls $R^6$ dasselbe wie unter b) angegeben bedeutet — Monoarylacetylene der Formel VII

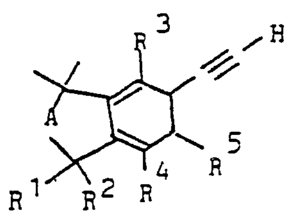

(VII)

worin $R^1$-$R^5$ und A die oben angegebene Bedeutung haben, mit einem Arylhalogenid der Formel VIII

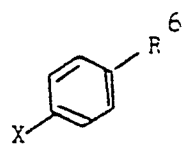

(VIII)

worin X ein Halogenatom bedeutet, in Gegenwart eines Katalysators, und einer Base umsetzt und die so erhaltenen Verbindungen gegebenenfalls nach Standardmethoden in weitere Verbindungen der Formel I umwandelt.

Die Halogenierung von Verbindungen der Formel II gemäß a) erfolgt zweckmäßigerweise mit Brom in einem Lösungsmittel bei Temperaturen bis zu 50 °C, vorzugsweise im Bereich von — 15 °C bis 0 °C. Als Lösungsmittel finden chlorierte Kohlenwasserstoffe, insbesondere Chloroform oder Tetrachlorkohlenstoff Verwendung. Anstelle von freiem Brom können auch Komplexe von molekularem Brom mit Kronenethern, z. B. Dibenzo-18-Krone-6, oder Perbromide wie z. B. Tetrabutylammoniumtribromid verwendet werden.

Geeignete Basen für die Eliminierung von zwei Moläquivalenten Bromwasserstoff aus den so erhaltenen Dibromverbindungen sind die Hydroxide, Alkoholate, Hybride und Amide der Alkali- und Erdalkalimetalle. Man arbeitet zweckmäßigerweise in einem Lösungsmittel ; in wäßrigen Lösungsmitteln und/oder bei Verwendung von Hydroxiden als Basen werden unter den für die Eliminierung üblichen Reaktionsbedingungen, d. h. bei einer Temperatur bis zu 200 °C, die Benzoesäuren der Formel I ($R^6$ = Carboxyl) durch Verseifung gebildet. Als besonders vorteilhaft hat sich das System Kaliumhydroxid in n-Butanol bei 120 °C erwiesen. Die Verseifung der Nitrilgruppe wird vermieden, wenn man unter Ausschluß von Hydroxylionen arbeitet, z. B. mit Kalium-tert.-butanolat als Base in Tetrahydrofuran oder Dimethylsulfoxid bei 25 °C bis 60 °C, oder besonders vorteilhaft in Petrolether in Gegenwart eines Phasen-Transfer-Katalysators, vorzugsweise 18-Krone-6, beim Siedepunkt des Reaktionsgemisches.

Die Verbindungen der Formel II sind in der DE-OS 3 202 125 beschrieben oder können nach den dort angegebenen Verfahren hergestellt werden.

Die Umsetzungen nach Wittig-Horner gemäß b) und c) verlaufen bei einer Temperatur bis zu 100 °C,

zweckmäßig bei 20 bis 50 °C. Die Umsetzungen können bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzungen in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylsulfoxid oder deren Mischungen, wobei die Umsetzung im allgemeinen bei einer Temperatur bis zu 30 °C abläuft.

Die Umsetzungen werden in Gegenwart eines Deprotonierungsmittels vorgenommen. Geeignet sind Alkalimetallhydride und Alkalimetallamide, insbesondere des Natriums und Kaliums, die Natrium- und Kaliumsalze von Dimethylsulfoxid, Alkyllithiumverbindungen wie n-Butyllithium oder Alkalimetallalkoholate, vorzugsweise Natriummethanolat, oder Kalium-tert.-butanolat.

Die Gesamtreaktion [b) und c)] (« Wittig-Horner-Reaktion » + Eliminierung) verläuft überraschenderweise besonders glatt mit 2 Moläquivalenten Kalium-tert.-butanolat in Dimethylsulfoxid als Lösungsmittel in einem Eintopfverfahren (vgl. J. Amer. Chem. Soc. 87, 2777 (1965)).

Bei Reaktion d) werden aus Verbindungen der Formel VII in üblicher Weise in situ die entsprechenden Kupferacetylide hergestellt, die mit den Arylhalogeniden VIII, vorzugsweise den Bromiden und -iodiden weiter zu Verbindungen der Formel I umgesetzt werden. Alternativ kann die Kupplungsreaktion, direkt von den Acetylenen VII ausgehend, durch Triphenylphosphinkomplexe des Palladiums und Nickels katalysiert werden. In allen Fällen ist die Gegenwart einer Base zweckmäßig, beispielsweise einer organischen Stickstoffbase, wie Triethylamin oder Pyridin, oder eines Alkalimetallalkoholats, wie Natriummethanolat oder Natriumphenolat. Gegebenenfalls wird in einem Lösungsmittel gearbeitet, vorzugsweise in Dimethylformamid oder Tetrahydrofuran. Die Umsetzung verläuft bei einer Temperatur von 50 bis 150 °C, zweckmäßigerweise bei 50 °C (Aryliodide) oder 100 °C (Arylbromide).

Die für die Verfahren b, c und d benötigten Ausgangsstoffe sind nach bekannten Verfahren erhältlich :

1-Aryl-1-chlormethylphosphonate der Formel III und der Formel V können z. B. dadurch hergestellt werden, daß man in an sich bekannter Weise den entsprechenden aromatischen Aldehyd mit einem Dialkylphosphit, gegebenenfalls in Gegenwart einer katalytischen Menge Base, z. B. Triethylamin oder Natriummethanolat oder besonders vorteilhaft Kaliumtert.-butanolat umsetzt ; die so hergestellten 1-Aryl-1-hydroxymethylphosphonate werden dann üblicherweise mit Thionylchlorid oder Phosphoroxytrichlorid, und falls zweckmäßig, in Gegenwart eines säurebindenden Mittels wie Pyridin oder Triethylamin, behandelt.

Die für die Wittig-Horner-Reaktion benötigten Aldehyde der Formel VI lassen sich beispielsweise durch Formylierung der entsprechenden Tetralin- oder Indanderivate in Gegenwart einer Lewis-Säure herstellen. Die Formylierung wird vorteilhaft mit Hexamethylentetramin/Trifluoressigsäure durchgeführt. Tetrahydrotetramethylnaphthalinderivate sind von T.F. Wood et al. in USP 3 442 640 und USP 3 499 751 beschrieben oder können nach dem dort angegebenen Verfahren aus 2,5-Dichlor-2,5-dimethylhexan und einem entsprechend substituierten Benzol durch Friedel-Crafts-Alkylierung hergestellt werden.

Die als Ausgangsprodukte verwendeten Monoarylacetylene der Formel VII lassen sich z. B. wie folgt herstellen :

Ein Arylmethylketon der Formel IX

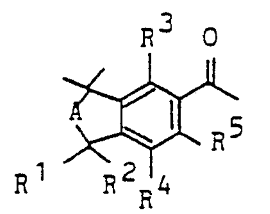

(IX)

worin A, $R^1$, $R^2$, $R^3$, $R^4$ und $R^5$ die genannten Bedeutungen haben, werden in an sich bekannter Weise mit Phosphorpentachlorid in Gegenwart einer Base wie z. B. Pyridin bei 0 bis 25 °C in die entsprechenden 1-Aryl-1-chlorethylene umgewandelt, welche mit einer Base, vorzugsweise Kalium-tert.-butanolat, in einem aprotisch dipolaren Lösungsmittel wie Dimethylsulfoxid bei 25 bis 40 °C zu den Monoarylacetylenen der Formel VII umgewandelt werden.

Die nach den oben genannten Verfahren a-d hergestellten Substanzen lassen sich anschließend wie folgt weiter abwandeln :

Die Benzoesäureester der Formel I ($R^6$ = Carboalkoxy) werden, falls erwünscht, durch Esterverseifung in die freien Carbonsäuren überführt. Umgekehrt läßt sich natürlich die freie Säure in bekannter Weise verestern.

Zweckmäßigerweise wird die Verseifung/Veresterung in Gegenwart eines Verdünnungs- oder Lösungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, oder in einem niederen aliphatischen Alkohol, wie Methanol, Ethanol, Propanol oder Isopropanol, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die Verseifung geschieht bevorzugt in Gegenwart von Alkali, wie Alkalimetallhydroxide, Alkalimetallcarbonate und -hydrogencarbonate, insbesondere des Natriums und Kaliums, organischen tertiären Basen, wie Pyridin oder niedere Trialkylamine, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die Veresterung geschieht vorteilhaft, indem man die Carbonsäure zunächst in ihr Salz überführt und dieses mit einem entsprechenden Alkylhalogenid, vorzugsweise einem Alkylbromid oder -iodid, behandelt. Als Deprotonierungsmittel für die Herstellung der Salze in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid und insbesondere Methylethylketon, wobei die Umsetzung beim Siedepunkt des Reaktionsgemisches durchgeführt wird.

Die erfindungsgemäßen Amide können in an sich bekannter Weise hergestellt werden, indem man die Benzoesäuren I ($R^6 = COOH$) zunächst in carbonylaktivere Derivate überführt, z. B. in die Säurehalogenide, -azide, -imidazolide, oder -anhydride, die O-Acyl-N,N'-dicyclohexylisoharnstoffe oder p-Nitrophenylester, und diese mit Aminen $HNR^{14}R^{15}$ behandelt. In den Fällen besonders reaktiver Amine, vor allem Ammoniak, ist die direkte Amidolyse von Estern (mit dem Rest $—OR^{13}$) bevorzugt.

Ein Halogenid der Benzoesäure I ($R^6 = COOH$), vorzugsweise das Säurechlorid, kann durch Reaktion mit 2-Aminoethanol und anschließende Cyclisierung in ein Oxazolinderivat der Formel (I) überführt werden.

Eine Carbonsäure, ein Carbonsäureester oder ein Carbonsäureamid der Formel I ($R^6 = COR^{11}$) kann in an sich bekannter Weise zu den entsprechenden Alkoholen bzw. Aminen reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetz, wie Diethylether, Dioxan oder Tetrahydrofuran. Führt man die Reduktion mit Diisobutylaluminiumhydrid oder einem Alkoxynatriumaluminiumhydrid durch, so ist die Verwendung von Kohlenwasserstoffen wie Hexan oder Toluol bevorzugt.

So erhaltene Amine oder Alkohole können in an sich bekannter Weise mit einem Alkanoylhalogenid oder -anhydrid, einem Aralkylhalogenid oder -anhydrid oder einem Aroylhalogenid oder -anhydrid zweckmäßigerweise in einem inerten Verdünnungs- oder Lösungsmittel, z. B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs- oder Lösungsmittel in die erfindungsgemäßen Amide und Ester der Formel (I) überführt werden. Bevorzugt werden die Umsetzungen in Gegenwart einer Base als säurebindendes Mittel in einem zwischen — 20 °C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

In ähnlicher Weise kann ein Alkohol I ($R^6 = CHR^7—OH$) mit einem Phosphorylhalogenid, vorzugsweise einem Phosphorylchlorid $Cl—P(O)(OR^{13})_2$ oder $Cl—P(O)(NR^{14}R^{15})_2$, oder einem Phosphorsäureanhydrid zum entsprechenden einem Phosphat oder Phosphorsäureamid umgesetzt werden. Zur Herstellung von Dihydrogenphosphaten ($R^{13} = H$) erwies sich das Trichlorimidat der Phosphorsäure als besonders vorteilhaftes Phosphorylierungsreagenz.

Ein unter die Formel I fallender Alkohol kann mit Alkylhalogeniden $R^{15}$-J, $R^{15}$-Br oder $R^{15}$-Cl in Gegenwart von Alkalimetallhydriden, vorzugsweise Natriumhydrid oder in Gegenwart von Alkyllithium-Verbindungen, vorzugsweise n-Butyllithium, in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen — 10 °C und 40 °C liegenden Temperaturbereich zum entsprechenden Ether umgesezt werden.

Ein unter die Formel I fallender Alkohol kann mit geeigneten Oxidationsmitteln, vorzugsweise Mangan(IV)-oxid, gegebenenfalls auf einem anorganischen Trägermaterial wie Silicagel oder Aluminiumoxid zum entsprechenden Aldehyd oxidiert werden. Vorteilhaft arbeitet man in einem inerten organischen Lösungsmittel, beispielsweise einem Kohlenwasserstoff wie Hexan oder in einem Ether wie beispielsweise

Tetrahydrofuran oder in Mischungen der gennanten Lösungs- und Verdünnungsmittel im Temperaturbereich zwischen — 10 °C und 30 °C. Die benötigte Reaktionszeit ist im wesentlichen von der Oxidationsaktivität des eingesetzten Mangan(IV)-oxids abhängig.

Einen Aldehyd I ($R^6$ = —CHO) kann man auch durch Reduktion des entsprechenden Nitrils mit Diisobutylaluminiumhydrid in einem Lösungsmittel vorzugsweise in Toluol, Hexan, Tetrahydrofuran oder Mischungen dieser Lösungsmittel in einem Temperaturbereich zwischen — 40 °C und Raumtemperatur erhalten.

Die unter die Formel I fallenden Aldehyde und Ketone werden auch dadurch erhalten, daß man ihre Ketale hydrolysiert, üblicherweise in Gegenwart einer Säure als Katalysator, vorzugsweise verdünnter Salz- oder Schwefelsäure, bei 20 °C oder bis zum Siedepunkt des Reaktionsgemisches erhöhter Temperatur. Zweckmäßigerweise arbeitet man in mit Wasser mischbaren Lösungsmitteln wie Aceton, Dioxan, Tetrahydrofuran, vorzugsweise in kurzkettigen Alkoholen wie Methanol und Ethanol.

Eine Carbonylverbindung der Formel I ($R^6$ = —$COR^{12}$) kann mit einer Phosphorverbindung der Formel (X) oder (XI)

$$(R^{21}O)_2\overset{O}{\overset{\|}{P}}CH_2CN \qquad (X)$$

$$(R^{21}O)_2\overset{O}{\overset{\|}{P}}CH_2COOR^{21} \qquad (XI)$$

in der $R^{21}$ die angegebene Bedeutung hat, in einer Wittig-Horner-Reaktion umgesetzt werden, zweckmäßigerweise arbeitet man in einem Lösungsmittel, vorzugsweise Tetrahydrofuran, Dimethylformamid oder Dimethylsulfoxid, und in Gegenwart der für solche Olefinierungen üblichen Basen wie z. B. Natriumhydrid oder Natriumethanolat. Die Umsetzung verläuft bei einer Temperatur bis zu 100 °C, zweckmäßig bei 20 bis 50 °C.

Die Nitril- bzw. Estergruppe wird anschließend, falls erwünscht, nach den vor- und nachstehend beschriebenen Methoden in andere funktionelle Gruppen umgewandelt.

Ein Nitril der Formel I ($R^6$ = —CN) kann in an sich bekannter Weise unter Säure- oder vorteilhafter Basenkatalyse zur entsprechenden Carbonsäure verseift werden. Als Basen bevorzugt sind Alkalimetallhydroxide, besonders Kaliumhydroxid, das im Überschuß eingesetzt wird. Als Lösungsmittel werden in der Regel mit Wasser mischbare Alkohole wie z. B. Methanol, Ethanol, Isopropanol oder n-Butanol eingesetzt. Die Umsetzung wird üblicherweise beim Siedepunkt des Reaktionsgemisches durchgeführt.

Aus den Nitrilen I ($R^6$ = —CN) können durch Addition eines Azids, z. B. eines Alkalimetallazids, vorzugsweise Natriumazid, in Gegenwart von Aluminiumchlorid oder Ammoniumchlorid die entsprechenden Tetrazole erhalten werden. Als Lösungsmittel verwendet man bevorzugt cyclische Ether, wie Dioxan oder Tetrahydrofuran, sowie insbesondere Dimethylformamid oder deren Mischungen, wobei die Umsetzung im allgemeinen bei einer Temperatur von 60-100 °C abläuft.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium-, Alkalimetall-, insbesondere des Natriums, Kaliums und Lithiums, Erdalkalimetallsalze, insbesondere des Calciums oder Magnesiums, sowie Salze mit geeigneten organischen Basen, wie mit niederen Alkylaminen, z. B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan, sowie mit Piperidin oder Morpholin.

Gegebenenfalls werden die erhaltenen erfindungsgemäßen Amine der Formel (I) nach bekannter Verfahrensweise in das Säureadditionssalz einer physiologisch verträglichen Säure überführt. Als übliche physiologisch verträgliche organische oder anorganische Säure kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band 10, Seiten 224-225, Birkhäuser Verlag, Basel und Stuttgart, 1966, entnommen werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische und therapeutische Behandlung von Praekanzerosen und Tumoren.

Die pharmakologischen Wirkungen können beispielsweise in den nachfolgenden Testmodellen aufgezeigt werden : die erfindungsgemäßen Verbindungen heben an Hamstertreachealgewebe in vitro

die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1976) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Esperimental Cell Research 117, 15-22 (1978) und Proc. Natl. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis-Modell bestimmt werden. Die dermatologische Aktivität, beispielsweise für die Behandlung von Akne, kann u. a. durch die komedolytische Aktivität und die Fähigkeit nachgewiesen werden, die Anzahl der Zysten im Modell der Rhino-Maus zu reduzieren.

Diese Methode ist von L. H. Kligman et al. in The Journal of Investigative Dermatology 73, 354-358 (1978) und von J. A. Mezick et al. in « Models of Dermatology » (Ed. Maibach, Lowe), Vol. 2, S. 59-63, Karger, Basel (1985) beschrieben.

Die Testsubstanz wurde in einem geeigneten Vehikel auf der gesamten Rückenpartie der Rhino-Maus topisch aufgetragen (100 µl), einmal täglich, an fünf aufeinanderfolgenden Tagen pro Woche, zwei Wochen lang. Ungefähr 72 h nach der letzten Behandlung wurde die dorsale Haut entfernt und in 0,5 %iger Essigsäure 18 h bei 4-6 °C belassen. Danach wurde eine ca. $2 \times 5\ cm^2$ große Fläche herausgeschnitten, die Epidermis abgeschält, auf einen Objektträger aufgebracht (mit der dermalen Seite nach oben) und mittels Alkohol/Xylol wasserfrei gespült, bis die Epidermis durchsichtig erscheint. Die Probe wurde durch Überziehen mit Permount fixiert und mikroskopisch ausgewertet. Gemessen wurde der Durchmesser von jeweils 10 Utriculi in jeweils 5 frei gewählten Feldern und daraus durch Vergleich mit der Utriculusdurchmessers berechnet. Die folgende Tabelle zeigt die erhaltenen Ergebnisse :

Tabelle

| Substanz | Dosis mg/ml | Reduktion des Ultriculusdurchmessers |
|---|---|---|
| 6 | 0,01 | 81,9 |
| | 0,001 | 70,9 |
| | 0,0001 | 51,3 |
| 14 | 0,02 | 66,9 |
| 12 | 2 | 78,0 |
| | 0,2 | 38,2 |
| 1 | 2 | 54,7 |

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,000001 bis 1 %iger Konzentration, bevorzugt in 0,00001 bis 0,1 %iger Konzentration und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Üblicherweise verwendete pharmazeutische technische Hilfsstoffe sind beispielsweise für die lokale

Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleichmittel usw. zugesetzt werden. Voraussetzung ist, daß alle bei der Herstellung pharmazeutischer Zubereitung verwendeten Stoffe toxikologisch unbedenklich sind und mit den verwendeten Wirkstoffen verträglich sind.

A. Synthese von Ausgangsprodukten

Allgemeine Arbeitsvorschrift zur Herstellung von 1-Chlor-1-arylmethylphosphonsäurediethylestern

Zu 152 g (1,1 mol) Diethylphosphit und 1 mol des entsprechenden aromatischen Aldehyds wurden 7,5 g (0,067 mol) Kalium-tert.-butanolat auf einmal zugegeben. Der darauffolgende Temperaturanstieg wurde mit Hilfe der Rührergeschwindigkeit so kontrolliert, daß die Temperatur nicht über den Bereich 70-90 °C hinausging. In manchen Fällen ist kurze externe Kühlung notwendig. Nachdem sich das Gemisch abgekühlt hatte, wurde es mit Wasser und Essigester verrührt. Die organische Phase wurde abgetrennt, über Natriumsulfat getrocknet und eingeengt. Der Rückstand wurde schließlich aus Petrolether oder Ether umkristallisiert, und man erhielt die reinen 1-Hydroxy-1-arylmethylphosphonsäurediethylester. Dieser wurde in die angegebene Menge Thionylchlorid portionsweise eingetragen, wobei die Temperatur auf ca. 35 °C anstieg. Man ließ 30 min. nachrühren und destillierte überschüssiges Thinoylchlorid im Vakuum ab. Durch Zugabe von Toluol und erneutes Abdestillieren wurde restliches Thinoylchlorid entfernt. Der so gewonnene rohe 1-Chlor-1-arylmethylphosphonsäurediethylester wurde wie jeweils angegeben gereinigt.

a) 1-Chlor-1-(4-tolyl) methylphosphonsäurediethylester

Aus 115,4 g (0,96 mol) 4-Tolylaldehyd wurden 177,8 g (72 %) 1-Hydroxy-1-(4-tolyl) methylphosphonsäurediethylester [vgl. Abramov, Zh. obshch. Chim. 27, 169, 172 (1957) ; CA 61, 12878 (1957)] erhalten, und aus 98 g (0,38 mol) der letztgenannten Verbindung und 188 ml Thionylchlorid wurden nach Destillation 73 g (66 %) der Titelverbindung gewonnen, Sdp. 139 °C (0,3 mbar).

b) 1-Chlor-1-(5,6,7,8-tetrahydro-5,5,8,8-tetramethyl-2-naphthyl)-methylphosphonsäurediethylester

Aus 216 g (1 mol) 2-Formyl-5,6,7,8-tetrahydro-5,5,8,8-tetramethylnaphthalin wurden 251 g (71 %) 1-Hydroxy-1-(5,6,7,8-tetrahydro-5,5,8,8-tetramethyl-2-naphthyl) methylphosphonsäurediethylester vom Schmp. 93-95 °C erhalten, und aus 203 g (0,57 mol) der letztgenannten Verbindung und 287 ml Thionylchlorid wurden 188,4 g (88 %) der Titelverbindung gewonnen. Da sich das Material beim Destillieren zersetzt, wurde die Aufarbeitung wie folgt abgeändert. Das nach Entfernen des Thionylchlorids erhaltene Rohprodukt wurde in Toluol gelöst und mit 5 g Kaliumcarbonat 20 min. gerührt. Man filtrierte vom Feststoff ab, entfernte das Solvens und zerkleinerte die erstarrte Masse in einer Reibschale. Das so gewonnene Material ist nach H-NMR-Spektrum ca. 85 %ig, Schmp. 65-66 °C.

Allgemeine Arbeitsvorschrift zur Herstellung von Monoacrylacetylenen

Zu einem auf 40 °C erwärmten Gemisch aus 260 g Phosphorpentachlorid, 350 ml Pyridin und 2,6 l Toluol wurde eine Lössung von 0,38 mol des entsprechenden Acetophenonderivates in 260 ml Pyridin zugetropft. Man rührte anschließend 3 h bei Rückflußtemperatur und 16 h bei Raumtemperatur. Dann wurde die Toluolphase abdekantiert, mit Wasser gewaschen (exotherm !), getrocknet ($Na_2SO_4$) und eingeengt. Der Rückstand wurde in 51 ml Dimethylsulfoxid gelöst. Zu dieser Lösung wurden bei 20-35 °C 28,6 g Kalium-tert.-butanolat in 120 ml Dimethylsulfoxid zugetropft. Man ließ 16 h bei Raumtemperatur nachrühren und goß dann auf Wasser. Man extrahierte dreimal mit Ether, wusch die vereinigten Etherphasen mit Wasser, trocknete ($Na_2SO_4$) und engte ein. Das rohe Acetylen wurde durch Destillation gereinigt.

Nach diesem Verfahren wurden folgende Verbindungen hergestellt :

(5,6,7,8-Tetrahydro-5,5,8,8-tetramethyl-2-naphthyl) ethin, Sdp. 82-94 °C (0,1 mbar), Ausbeute 9 %, aus 2-Acetyl-5,6,7,8-tetrahydro-5,5,8,8-tetramethylnaphthalin.

(1,2-Dihydro-1,1,2,3,3-pentamethyl-5(1H)-indenyl) ethin, Sdp. 85-90 °C (0,3 mbar), Ausbeute 27 %, aus 5-Acetyl-1,2-dihydro-1,1,2,3,3-pentamethyl-(1H)-inden.

(1,2-Dihydro-1,1,3,3-tetramethyl-5(1H)-indenyl) ethin, Sdp. 94 °C (2 mbar), Ausbeute 9%, aus 5-Acetyl-1,2-dihydro-1,1,3,3-tetramethyl-(1H)-inden.

(5,6,7,8-Tetrahydro-3,8,8-trimethyl-2-naphthyl) ethin, Sdp. 100-105 °C (1 mbar), Ausbeute 12 %, aus 2-Acetyl-5,6,7,8-tetrahydro-3,8,8-trimethylnaphthalin.

B. Synthese der erfindungsgemäßen Verbindungen

Beispiel 1

4-[(5,6,7,8-Tetrahydro-5,5,8,8-tetramethyl-2-naphthyl) ethinyl] benzonitril

a) 6,7 g (0,014 mol) 1,2-Dibrom-1-(4-cyanophenyl)-2-(5,6,7,8-tetrahydro-5,5,8,8-tetramethyl-2-naphthyl)-ethan (siehe Beispiel 6a) wurden in 26 ml Petrolether suspendiert. Nach Zugabe von 3,2 g (0,028 mol) Kalium-tert.-butanolat stieg die Temperatur des Reaktionsgemisches auf 50 °C an. Man erhitzte 1 h unter Rückfluß, setzte 20 mg (0,1 mol) 18-Krone-6 zu und erhitzte weitere 10 h unter Rückfluß. Danach wurde das Gemisch auf 500 ml Eiswasser gegossen und zweimal mit Petrolether extrahiert. Die organische Phase wurde mit Wasser gewaschen, über Natriumsulfat getrocknet, und nach Einengen im Vakuum erhielt man 4,3 g rohes Produkt. Umkristallisation aus Ethanol ergab 1,9 g (43 %) der Titelverbindung, Schmp. 166 °C.

b) Zu einer Lösung von 22,6 g (0,06 mol) 1-Chlor-1-(5,6,7,8-tetrahydro-5,5,8,8-tetramethyl-2-naphthyl)-methylphosphonsäurediethylester (ca. 85 %ig) und 7,86 g (0,06 mol) 4-Cyanobenzaldehyd in 190 ml trockenem Dimethylsulfoxid wurde bei Raumtemperatur eine Lösung von 13,8 g (0,123 mol) Kalium-tert.-butanolat in 65 ml Dimethylsulfoxid innerhalb 30 min. zugetropft. Man ließ 1 h nachrühren, goß danach auf 1 l Eiswasser, säuerte mit wenig verdünnter Salzsäure an und saugte das entstandene Kristallisat ab. Nach Umkristallisieren aus Ethanol wurden 9,2 g (49 %) der Titelverbindung, Schmp. 157 °C erhalten.

c) Ein Gemisch aus 4 g (19 mmol) (5,6,7,8-Tetrahydro-5,5,8,8-tetramethyl-2-naphthyl) ethin, 2,8 g (12,5 mmol) 4-Brombenzonitril, 50 mg Palladium(II)-acetat, 100 mg Triphenylphosphin und 25 ml wasserfreies entgastes Triethylamin wurde 4 h unter Stickstoff und unter Rückfluß erhitzt. Man filtrierte danach vom Feststoff ab und engte das Filtrat ein.

Der Rückstand ergab nach Umkristallisation 2,6 g (66 %) der Titelverbindung, die ohne weitere Reinigung zur Carbonsäure verseift wurden (s. Beispiel 6c).

## Beispiel 2

4-[(1,2-Dihydro-1,1,2,3,3-pentamethyl-5(1H)-indenyl) ethinyl]-benzonitril

Analog dem in Beispiel 1c beschriebenen Verfahren erhielt man aus 4 g (19 mmol) (1,2-Dihydro-1,1,2,3,3-pentamethyl-5(1H)-indenyl)-ethin und 2,8 g (12,5 mmol) 4-Brombenzonitril 2 g (51 %) der Titelverbindung nach Umkristallisation aus Isopropanol, Schmp. 110-112 °C, wobei der Rückstand des Filtrats mit Natriumhydrogencarbonatlösung/Methylenchlorid extrahiert und wie üblich weiterbehandelt wurde.

## Beispiel 3

4-[(1,2-Dihydro-1,1,3,3-tetramethyl-5(1H)-indenyl) ethinyl]-benzonitril

Analog dem in Beispiel 1c beschriebenen Verfahren erhielt man aus 3,7 g (19 mmol) (1,2-Dihydro-1,1,3,3-tetramethyl-5(1H)-indenyl)-ethin und 2,8 g (12,5 mmol) 4-Brombenzonitril 1,4 g (37 %) der Titelverbindung nach Umkristallisation aus Ethanol, die ohne weitere Reinigung zur Carbonsäure verseift wurden (s. Beispiel 6c).

## Beispiel 4

4-[(5,6,7,8-Tetrahydro-3,8,8-trimethyl-2-naphthyl) ethinyl]-benzonitril

Analog dem in Beispiel 1c beschriebenen Verfahren erhielt man aus 4,4 g (22 mmol) (5,6,7,8-Tetrahydro-3,8,8-trimethyl-2-naphthyl) ethin und 2,7 g (15 mmol) 4-Brombenzonitril 1,3 g (29 %) der Titelverbindung nach Umkristallisation aus Ethanol, Schmp. 128-130 °C.

## Beispiel 5

4-[(5,6,7,8-Tetrahydro-3-methoxy-5,5,8,8-tetramethyl-2-naphthyl) ethinyl]-benzonitril

Analog dem in Beispiel 1a beschriebenen Verfahren erhielt man aus 34 g (0,1 mol) 4-[2-(5,6,7,8-Tetrahydro-3-methoxy-5,5,8,8-tetramethyl-2-naphthyl)-1-ethenyl] benzonitril 6,9 g (20 %) Rohprodukt, aus dem durch Umkristallisation 2,6 g der Titelverbindung gewonnen wurden, Schmp. 165-167 °C.

## Beispiel 6

4-[(5,6,7,8-Tetrahydro-5,5,8,8-tetramethyl-2-naphthyl) ethinyl] benzoesäure

a) Zu einer Suspension von 26,5 g (0,08 mol) (E)-4-[2-(5,6,7,8-Tetrahydro-5,5,8,8-tetramethyl-2-naphthyl)-1-ethenyl] benzonitril in 120 ml Chloroform wurde bei − 15 bis 10 °C eine Lösung von 148 g (0,09 mol, 4,7 ml) Brom in 25 ml Chloroform innerhalb 20 min. zugetropft. Man ließ 15 min. nachrühren, engte das Reaktionsgemisch am Rotationsverdampfer ein und kristallisierte den Rückstand aus Methanol um. Man erhielt 26,7 g (70 %) 1,2-Dibrom-1-(4-Cyanophenyl)-2-(5,6,7,8-tetrahydro-5,5,8,8-tetramethyl-2-naphthyl)-ethan als Gemisch der Diastereomeren vom Schmp. 174 °C bis 175 °C.

50,7 g (0,11 mol) dieser Verbindungen wurden zu einer alkalischen Lösung von 60,2 g Kaliumhydroxid in 143 ml n-Butanol gegeben und 1 h unter Rückfluß erhitzt. Das abgekühlte Reaktionsgemisch wurde auf 1,5 l Eiswasser gegeben und mit konz. Salzsäure angesäuert. Der ausgefallene Niederschlag wurde abgesaugt, mit Wasser, dann mit Methanol gewaschen und im Stickstoffstrom getrocknet. Nach Umkristallisation aus Isopropanol erhielt man 26,9 g (74 %) der Titelverbindung, Schmp. 265-266 °C.

b) 22,6 g (0,06 mol) 1-Chlor-1-(5,6,7,8-tetrahydro-5,5,8,8-tetramethyl-2-naphthyl)-methylphosphonsäurediethylester (ca. 85 %ig) und 9 g 4-Carboxybenzaldehyd wurden in 190 ml trockenem Dimethylsulfoxid vorgelegt.

Bei Raumtemperatur wurden unter Rühren 21 g (0,185 mol) Kalium-tert.-butanolat in 65 ml Dimethylsulfoxid zugetropft. Man ließ 1 h nachrühren. Danach wurde das Reaktionsgemisch auf 1 l Eiswasser gegossen und mit 20 %iger Schwefelsäure angesäuert. Der entstandene Niederschlag wurde abgesaugt, mit Wasser gewaschen, und aus Isopropanol umkristallisiert. Man erhielt 14 g (70 %) der Titelverbindung. Weiteres zweimaliges Umkristallisieren ergab 6 g reines Material, Schmp. 263-264 °C.

c) 2,6 g (8 mmol) 4-[(5,6,7,8-Tetrahydro-5,5,8,8-tetramethyl-2-naphthyl)-ethinyl] benzonitril (Beispiel 1) und 4,6 g Kaliumhydroxid (85 %ig) wurden in 17 ml n-Butanol 1,5 h unter Rückfluß erhitzt. Das abgekühlte Reaktionsgemisch wurde in 100 ml Wasser gelöst und dreimal mit Ether extrahiert. Die wäßrige Phase wurde bei vermindertem Druck von Etherresten befreit und mit 2 N HCl angesäuert. Man saugte den ausgefallenen Niederschlag ab, wusch ihn mit Wasser und trocknete ihn im Stickstoffstrom. Zurück blieben 2,1 g Rohprodukt, Umkristallisation aus Isopropanol erbrachte 1,2 g (44 %) der Titelverbindung, Schmp. 252-256 °C, Reinheit nach HPLC 99,9 % (C 18 reversed phase ; Acetonitril/$H_2O$ 9 : 1 + 0,1 % Essigsäure ; 100 ml/min ; $t_R$ : 7 min).

Beispiel 7

4-[(5,6,7,8-Tetrahydro-3-methoxy-5,5,8,8-tetramethyl-2-naphthyl)-ethinyl] benzoesäure

Analog dem in Beispiel 6c beschriebenen Verfahren erhielt man aus 1 g (2 mmol) 4-[(5,6,7,8-Tetramethyl-3-methoxy-5,5,8,8-tetramethyl-2-naphthyl)-ethinyl] benzonitril (Beispiel 5) 0,5 g (64 %) der Titelverbindung, Schmp. 231-234 °C, wobei die Etherextraktion entfiel und das rohe Kristallisat aus Methanol umkristallisiert wurde.

Beispiel 8

4-[(1,2-Dihydro-1,1,2,3,3-pentamethyl-5(1H)-indenyl) ethinyl]-benzoesäure

Analog dem in Beispiel 6c beschriebenen Verfahren erhielt man aus 2 g (6 mmol) 4-[(1,2-Dihydro-1,1,2,3,3-pentamethyl-5(1H)-indenyl) ethinyl] benzonitril (Beispiel 2) 1,1 g (55 %) der Titelverbindung, Schmp. 267-270 °C, wobei die Etherextraktion und die Umkristallisation entfielen.

Beispiel 9

4-[(1,2-Dihydro-1,1,3,3-tetramethyl-5(1H)-indenyl) ethinyl]-benzoesäure

Analog dem in Beispiel 6c beschriebenen Verfahren erhielt man aus 1,4 g (5 mmol) 4-[(1,2-Dihydro-1,1,3,3-tetramethyl-5(1H)-indenyl) ethinyl] benzonitril (Beispiel 3) 0,9 g (60 %) der Titelverbindung, Schmp. 236 °C, wobei die Etherextraktion entfiel und das rohe Kristallisat aus Isopropanol umkristallisiert wurde.

Beispiel 10

4-[(5,6,7,8-Tetrahydro-3,8,8-trimethyl-2-naphthyl) ethinyl] benzoesäure

1 g (3,3 mol) 4-[(5,6,7,8-Tetrahydro-3,8,8-trimethyl-2-naphthyl)-ethinyl] benzonitril (Beispiel 4) in 19 ml Ethanol und 19 ml 10 n NaOH wurden 6,5 h unter Rückfluß erhitzt. Nach dem Abkühlen goß man auf Wasser, säuerte mit 2 N HCl an, saugte das ausgefallene Kristallisat ab, wusch mit Wasser nach und erhielt nach Trocknen 1 g (95 %) der Titelverbindung, Schmp. 203-206 °C.

Beispiel 11

4-[(5,6,7,8-Tetrahydro-5,5,8,8-tetramethyl-2-naphthyl) ethinyl] benzoesäure-ethylester

3,0 g (9 mmol) 4-[(5,6,7,8-Tetrahydro-5,5,8,8-tetramethyl-2-naphthyl) ethinyl] benzoesäure, 4 g (30 mmol) Kaliumcarbonat und 2,9 g (18,9 mmol) Iodethan wurden in 27 ml 2-Butanon bis zur vollständigen Umsetzung (DC-Kontrolle) unter Rückfluß erhitzt. Nach dem Abkühlen wurde vom Feststoff abfiltriert

und das Filtrat eingeengt. Der Rückstand wurde aus Methanol umkristallisiert. Man erhielt 2,1 g (65 %) der Titelverbindung, Schmp. 137-138 °C.

Beispiel 12

2-(5,6,7,8-Tetrahydro-5,5,8,8-tetramethyl-2-naphthyl)-1-[4-(1H-tetrazolyl-5-yl) phenyl] acetylen

2,15 g (0,033 mol) Natriumazid, 1,77 g (0,033 mol) Ammoniumchlorid und 9,2 g (0,03 mol) 4-[(5,6,7,8-Tetrahydro-5,5,8,8-tetramethyl-2-naphthyl) ethinyl] benzonitril wurden in 30 ml absolutem Dimethylformamid 12 h bei 120 °C gerührt. Dann wurde das abgekühlte Reaktionsgemisch auf 0,5 l Wasser gegossen und mit wenig verdünnter Salzsäure angesäuert. Das ausgefallene Kristallisat wurde abgesaugt und auf der Nutsche mehrmals mit Wasser und anschließend mit Methanol gewaschen, scharf abgesaugt und im Stickstoffstrom getrocknet. Man erhielt 9,0 g (84 %) der Titelverbindung, Schmp. 227-228 °C.

Beispiel 13

(5,6,7,8-Tetrahydro-3-methoxy-5,5,8,8-tetramethyl-2-naphthyl)-(4-tolyl)-acetylen

Zu einer Lösung von 23,2 g (0,09 mol) 1-Chlor-1-(4-tolyl)-methylphosphonsäurediethylester und 22,1 g (0,09 mol) 2-Formyl-5,6,7,8-tetrahydro-3-methoxy-5,5,8,8-tetramethyl-naphthalin in 270 ml trockenem Dimethylsulfoxid wurde bei Raumtemperatur eine Lösung von 20,2 g (0,18 mol) Kalium-tert.-butanolat in 45 ml Dimethylsulfoxid zugetropft. Man ließ 30 min. nachrühren, goß auf 1 l Eiswasser und extrahierte dreimal mit Ether. Die Etherphase wurde zweimal mit Wasser gewaschen, über Natriumsulfat getrocknet und eingeengt. Es blieben 22,6 g (76 %) leicht verunreinigtes Produkt zurück. Umkristallisation aus Methanol ergab 15,2 g (51 %) reines Material der Titelverbindung, Schmp. 127 °C.

Beispiel 14

(5,6,7,8-Tetrahydro-5,5,8,8-tetramethyl-2-naphthyl)-(4-tolyl)-acetylen

Analog dem in Beispiel 13 beschriebenen Verfahren erhielt man aus 61,4 g (0,22 mol) 1-Chlor-1-(4-tolyl)-methylphosphonsäurediethylester, 51,1 g (0,22 mol) 2-Formyl-5,6,7,8-tetrahydro-5,5,8,8-tetramethylnaphthalin und 50 g (0,44 mol) Kalium-tert.-butanolat nach 1 h Reaktionszeit 37,1 g (56 %) der Titelverbindung, Schmp. 99 °C, wobei das Reaktionsgemisch auf Wasser gegossen, angesäuert, und der ausgefallene Feststoff abgesaugt und zweimal aus Methanol umkristallisiert wurde.

Beispiel 15

(3-Fluor-5,6,7,8-tetrahydro-5,5,8,8-tetramethyl-2-naphthyl)-(4-tolyl)-acetylen

Analog dem in Beispiel 13 beschriebenen Verfahren erhielt man aus 27,7 g (0,1 mol) 1-Chlor-1-(4-tolyl)-methylphosphonsäurediethylester, 23,4 g (0,1 mol) 3-Fluor-2-formyl-5,6,7,8-tetrahydro-5,5,8,8-tetramethyl-naphthalin und 22,5 g (0,2 mol) Kalium-tert.-butanolat nach 1 h Reaktionszeit nach Umkristallisation aus Isopropanol 16,4 g (51 %) der Titelverbindung, Schmp. 60-61 °C.

Beispiel 16

(3-Ethyl-5,6,7,8-tetrahydro-5,5,8,8-tetramethyl-2-naphthyl)-(4-tolyl)-acytelen

Analog dem in Beispiel 13 beschriebenen Verfahren erhielt man aus 27,7 g (0,1 mol) 1-Chlor-(4-tolyl)-methylphosphonsäurediethylester, 23,4 g (0,1 mol) 3-Ethyl-2-formyl-5,6,7,8-tetrahydro-5,5,8,8-tetramethyl-naphthalin und 22,5 g (0,2 mol) Kalium-tert.-butanol nach 1 h Reaktionszeit 8,3 g (25 %) der Titelverbindung, Schmp. 72-73 °C, wobei das Reaktionsgemisch auf Wasser gegossen, angesäuert, der ausgefallene Niederschlag abgesaugt und aus Methanol und noch einmal aus Isopropanol umkristallisiert wurde.

Beispiel 17

4-[(5,6,7,8-Tetrahydro-5,5,8,8-tetramethyl-2-naphthyl)-ethinyl]-benzamid

Ein Gemisch aus 2,5 g (8 mmol) 4-[(5,6,7,8-Tetrahydro-5,5,8,8-tetramethyl-2-naphthyl) ethinyl] benzonitril (Beispiel 1), 60 ml tert.-Butanol und 7,5 g Kaliumhydroxidpulver wurden 4 h unter Rückfluß erhitzt. Nach dem Abkühlen goß man auf gesättigte Natriumchloridlösung und extrahierte zweimal mit Ether. Die Etherphasen wurden mit Natriumchloridlösung gewaschen, getrocknet ($Na_2SO_4$) und eingeengt. Zurück blieben 2,2 g (83 %) der reinen Titelverbindung, Schmp. 220-223 °C.

Beispiel 18

4-[(5,6,7,8-Tetrahydro-5,5,8,8-tetramethyl-2-naphthyl)-ethinyl]-benzaldehyd

Zu einer Lösung von 10 g (32 mmol) 4-[(5,6,7,8-Tetrahydro-5,5,8,8-tetramethyl-2-naphthyl) ethinyl] benzonitril (Beispiel 1) in 120 ml abs. Ether gab man 56 ml (67 mmol) einer 20 %igen Diisobutylaluminiumhydridlösung in Hexan. Man ließ 40 min. nachrühren, tropfte 150 ml gesättigte Weinsäurelösung zu und ließ 1 h nachrühren. Dann extrahierte man dreimal mit Ether, die vereinigten Etherphasen wurden zweimal mit Wasser gewaschen, getrocknet ($Na_2SO_4$) und eingeengt. Aus dem Rückstand wurden durch Umkristallisation aus Isopropanol 3,8 g (38 %) der Titelverbindung erhalten, Schmp. 130 °C.

Beispiel 19

4-[(2,3-Dihydro-1,1,2,3,3-pentamethyl-5(1H)-indenyl) ethinyl]-benzaldehyd

Analog der in Beispiel 1c beschriebenen Arbeitsweise erhielt man aus 8 g (40 mmol) (2,3-Dihydro-1,1,2,3,3-pentamethyl-5(1H)-indenyl)ethin und 4,6 g (25 mmol) 4-Brombenzaldehyd nach Einengen des Filtrats einen Rückstand, der mit Natriumhydrogencarbonatlösung/Methylenchlorid extrahiert wurde. Aus dem Rückstand erhielt man durch Verrühren mit Methylenchlorid und wenig Cyclohexan 2,3 g (29 %) der Titelverbindung, Schmp. 106-107 °C.

Beispiel 20

4-[(5,6,7,8-Tetrahydro-5,5,8,8-tetramethyl-2-naphthyl) ethinyl]-benzylalkohol

Zu einer Suspension aus 1,9 g (49 mmol) Lithiumaluminiumhydrid in 150 ml abs. Ether wurde eine Suspension aus 15,8 g (48 mmol) 4-[(5,6,7,8-Tetrahydro-5,5,8,8-tetramethyl-2-naphthyl)-ethinyl] benzoesäure (Beispiel 6) in 160 ml abs. Ether zugetropft. Man rührte anschließend 3 h unter Rückfluß. Dann gab man nacheinander 50 ml Essigester, 200 ml Wasser und 150 ml 2N HCl tropfenweise zu und trennte die Phasen. Die wäßrige Phase wurde noch einmal mit Ether extrahiert, die vereinigten Etherextrakte wurden mit Wasser gewaschen, getrocknet ($Na_2SO_4$) und eingeengt. Das zurückbleibende Öl (17 g) wurde mit Heptan verrührt. Das entstandene Kristallisat wurde abgesaugt und getrocknet. Man erhielt so 7,2 g (48 %) der Titelverbindung, Schmp. 115-117 °C.

Beispiel 21

4-[(5,6,7,8-Tetrahydro-5,5,8,8-tetramethyl-2-naphthyl)-ethinyl]-benzylmethylether

Zu einer Suspension von 0,4 g (13 mmol) Natriumhydrid in 15 ml abs. Dimethylformamid wurde bei Raumtemperatur eine Lösung von 2 g (6,3 mmol) des im vorangegangenen Beispiel 20 beschriebenen Benzylalkoholderivats in 10 ml abs. Dimethylformamid zugetropft. Man ließ 1 h nachrühren und tropfte dann 1,5 g (10 mmol) Iodmethan zu. Man erhitzte 15 h bei 60 °C. Nach dem Abkühlen goß man auf Wasser und saugte den ausgefallenen Feststoff ab. Nach Umkristallisation aus Methanol erhielt man 0,8 g (38 %) der Titelverbindung, Schmp. 93-94 °C.

Beispiel 22

4-[(5,6,7,8-Tetrahydro-5,5,8,8-tetramethyl-2-naphthyl) ethinyl]-benzylacetat

Zu einem Gemisch aus 1,5 g (4,7 mmol) 4-[(5,6,7,8-Tetrahydro-5,5,8,8-tetramethyl-2-naphthyl) ethinyl] benzylalkohol (Beispiel 20) und 8,7 ml Pyridin wurden 1,7 ml Essigsäureanhydrid zugegeben. Man ließ 16 h bei Raumtemperatur rühren, goß danach auf Eis/Wasser und säuerte an. Der ausgefallene Feststoff wurde abgesaugt, mit Wasser gewaschen und getrocknet. Man erhielt so 1,3 g (77 %) der Titelverbindung, Schmp. 136-139 °C.

Beispiel 23

4-[(5,6,7,8-Tetrahydro-5,5,8,8-tetramethyl-2-naphthyl) ethinyl]-benzylamin

Zu einer Suspension von 2,8 g (73 mmol) Lithiumaluminiumhydrid in 150 ml abs. Ether wurde bei Raumtemperatur innerhalb 25 min. eine Lösung von 8,2 g (26 mmol) 4-[(5,6,7,8-Tetrahydro-5,5,8,8-tetramethyl-2-naphthyl)-ethinyl] benzonitril (Beispiel 1) in 150 ml abs. Ether zugetropft. Man rührte 3,5 h unter Rückfluß. Nach dem Abkhülen versetzte man vorsichtig mit Wasser, tropfte Natriumsulfatlösung zu

und trennte die Phasen. Die wüßrige Phase wurde noch zweimal mit Ether extrahiert, die vereinigten Etherphasen wurden mit Wasser einmal gewaschen, getrocknet ($Na_2SO_4$) und eingeengt. Zurück blieben 7,7 g (93 %) der Titelverbindung, Schmp. 84-88 °C.

Beispiel 24

N-Acetyl-4-[(5,6,7,8-Tetrahydro-5,5,8,8-tetramethyl-2-naphthyl) ethinyl]-benzylamin

Zu einem Gemisch aus 3,2 g (10 mmol) des im vorangegangenen Beispiel 23 beschriebenen Benzylaminderivates und 20 ml Pyridin wurden bei 0 °C 2,5 g (25 mmol) Essigsäureanhydrid zugetropft. Man goß anschließend auf Eis/Wasser, säuerte mit 0,5 N HCl an und saugte den ausgefallenen Feststoff ab. Nach Trocknen erhielt man 3,1 g (86 %) der Titelverbindung, Schmp. 220-223 °C.

Beispiel 25

(E)-4-[(5,6,7,8-Tetrahydro-5,5,8,8-tetramethyl-2-naphthyl) ethinyl] zimtsäure

Analog der in Beispiel 6b beschriebenen Arbeitsweise erhielt man aus 16,5 g (44 mmol) 1-Chlor-1-(5,6,7,8-Tetrahydro-5,5,8,8-tetramethyl-2-naphthyl) methylphosphonsäurediethylester (ca. 85 %ig), 7,8 g (44 mmol) 4-Formylzimtsäure und 15,5 g (137 mmol) Kalium-tert.-butanolat 6,2 g (39 %) der Titelverbindung, Schmp. 256-258 °C (aus Ethanol).

Beispiel 26

(E)-4-[(5,6,7,8-Tetrahydro-5,5,8,8-tetramethyl-2-naphthyl)-ethinyl] zimtsäurethylester

Analog der in Beispiel 11 beschriebenen Arbeitsweise erhielt man aus 2 g (5,5 mmol) der im vorangegangenen Beispiel 25 beschriebenen Zimtsäure, 2,6 g Kaliumcarbonat und 1,8 g Iodethan 2 g (93 %) der Titelverbindung, Schmp. 116-118 °C, wobei man das Reaktionsgemisch auf Wasser goß, den ausgefallenen Feststoff absaugte, mit wenig Methanol wusch und trocknete.

**Patentansprüche** (für die Vertragsstaaten : BE, CH, DE, FR, GB, IT, LI, NL, SE)

1. Diarylacetylene der Formel I

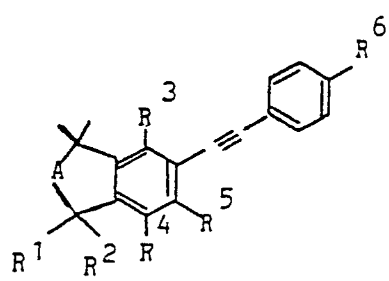

(I)

in der

$R^1$ und $R^2$ Wasserstoffatome oder Methylgruppen

$R^3$ ein Wasserstoffatom oder eine Methyl-, Hydroxy- oder $C_{1-6}$-Alkoxygruppe

$R^4$ ein Wasserstoffatom oder eine Methyl- oder Methoxygruppe

$R^5$ ein Wasserstoff- oder Halogenatom oder eine Methoxy- oder $C_{1-4}$-Alkylgruppe

A einen gegebenenfalls mit $C_{1-4}$-Alkylgruppen substituierten Methylen- oder Ethylenrest oder die Gruppen —CH=CH—, —CHOH—$CH_2$— oder —CO—$CH_2$— und

$R^6$ ein Wasserstoffatom, eine Methyl- oder Nitrilgruppe, den Tetrazolyl- oder 2-Oxazolinylrest, eine $C_{2-10}$-Ketalgruppe oder die Reste —$CHR^7$—$OR^8$, —$CHR^7$—$NR^9R^{10}$, —$COR^{11}$, —$CR^{12}$=CH—$COOR^{13}$ oder —$CR^{12}$=CH—CO—$NR^{14}R^{15}$ bedeuten, worin

$R^7$ ein Wasserstoffatom oder eine $C_{1-4}$-Alkylgruppe

$R^8$ ein Wasserstoffatom, eine $C_{1-4}$-Alkyl-, $C_{1-20}$-Alkanoyl-, eine gegebenenfalls substituierte Benzoylgruppe oder die Reste —P(O) $(OR^{13})_2$ oder —P(O) $(^{N4}R^{15})_2$ (mit $R^{13}$ in der Bedeutung eines Wasserstoffatoms, einer gegebenenfalls durch eine Hydroxygruppe substituierten $C_{1-8}$-Alkylgruppe, einer gegebenenfalls substituierten Aryl- oder gegebenenfalls im Arylteil substituierten Aralkygruppe, und mit $R^{14}$ und $R^{15}$ in der Bedeutung von Wasserstoffatomen, gegebenenfalls durch eine Hydroxygruppe substituierten $C_{1-6}$-Alkylgruppen, gegebenenfalls substituierten Aralkyl- oder Arylgruppen, oder $R^{14}$

und $R^{15}$ zusammen mit dem Stickstoffatom, an das sie gebunden sind, in der Bedeutung eines heterocyclischen Restes

$R^9$ und $R^{10}$ Wasserstoffatome, $C_{1-4}$-Alkyl-, $C_{1-6}$-Alkanoyl- oder gegebenenfalls substituierte Benzoylgruppen oder zusammen mit dem Stickstoffatom, an das sie gebunden sind, einen heterocyclischen Rest

$R^{11}$ ein Wasserstoff- oder Halogenatom, eine $C_{1-4}$-Alkylgruppe oder die Reste —$NR^{14}R^{15}$ oder —$OR^{13}$ (mit $R^{13}$, $R^{14}$ und $R^{15}$ in der oben angegebenen Bedeutung) und

$R^{12}$ ein Wasserstoffatom oder eine Methylgruppe darstellen,

sowie gegebenenfalls deren physiologisch verträgliche Salze.

2. 4-[(5,6,7,8-Tetrahydro-5,5,8,8-tetramethyl-2-naphthyl) ethinyl] benzoesäure.

3. 4-[(5,6,7,8-Tetrahydro-5,5,8,8-tetramethyl-2-naphthyl) ethinyl]-benzonitril.

4. 4-[(5,6,7,8-Tetrahydro-5,5,8,8-tetramethyl-2-naphthyl) ethinyl] benzoesäureethylester.

5. 2-(5,6,7,8-Tetrahydro-5,5,8,8-tetramethyl-2-naphthyl)-1 [4-(4-(1H-tetrazolyl-5-yl)-phenyl] acetylen.

6. (5,6,7,8-Tetrahydro-5,5,8,8-tetramethyl-2-naphthyl)-(4-tolyl)-acetylen.

7. 4-[(5,6,7,8-Tetrahydro-5,5,8,8-tetramethyl-2-naphthyl) ethinyl]-benzaldehyd.

8. 4-[(5,6,7,8-Tetrahydro-5,5,8,8-tetramethyl-2-naphthyl) ethinyl]-benzylalkohol.

9. Verfahren zur Herstellung der Diarylacetylene der Formel I gemäß Anspruch 1, dadurch gekennzeichnet, daß man

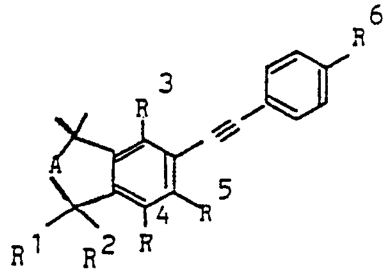

(I)

a) falls $R^6$ eine Cyanogruppe bedeutet — Stilbene der Formel II

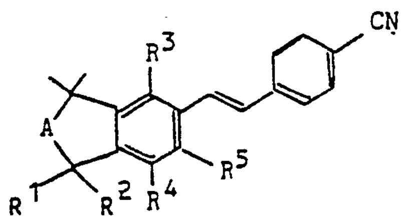

(II)

in der $R^1$-$R^5$ und A die oben angegebene Bedeutung haben, halogeniert und anschließend 2 Mole Halogenwasserstoff abspaltet, oder

b) falls $R^6$ ein Wasserstoffatom, eine Carboxyl-, Nitril- oder Formylgruppe bedeutet — ein α-Chlorbenzylphosphonat der Formel III

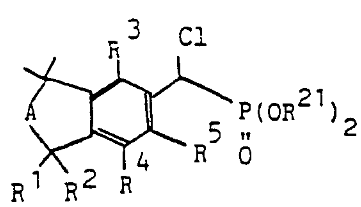

(III)

in der $R^1$-$R^5$ und A die oben angegebene Bedeutung haben, und $R^{21}$ eine $C_{1-3}$-Alkylgruppe bedeutet, mit Aldehyden der Formel IV

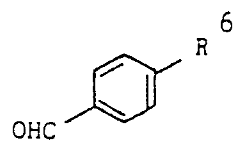

(IV)

umsetzt oder

c) falls $R^6$ eine Methyl- oder Nitrilgruppe oder einen 1,3-Dioxolan-2-yl-rest bedeutet — ein α-Chlorbenzylphosphonat der Formel V,

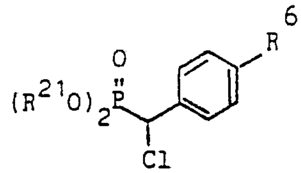

(V)

worin $R^{21}$ die oben angegebene Bedeutung hat, mit Aldehyden der Formel VI

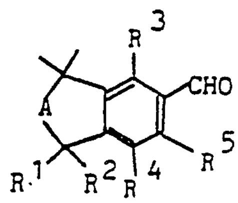

(VI)

worin $R^1$-$R^5$ und A die oben angegebene Bedeutung haben, umsetzt oder

d) falls $R^6$ dasselbe wie unter b) angegeben bedeutet — Monoarylacetylene der Formel VII

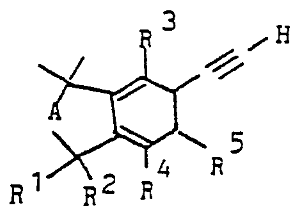

(VII)

worin $R^1$-$R^5$ und A die oben angegebene Bedeutung haben, mit einem Arylhalogenid der Formel VIII

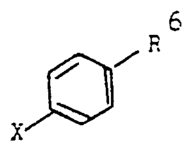

(VIII)

in Gegenwart eines Katalysators, und einer Base umsetzt und die so erhaltenen Verbindungen gegebenenfalls nach Standardmethoden in weitere Verbindungen der Formel I umwandelt.

10. Verwendung der Arylacetylene der Formel I gemäß Anspruch 1 zur Verwendung bei der Bekämpfung von Krankheiten.

**Patentansprüche** (für den Vertragsstaat AT)

1. Verfahren zur Herstellung der Diarylacetylene der Formel I

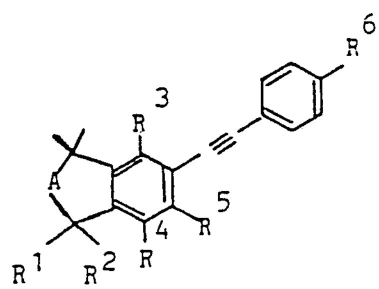

(I)

in der

$R^1$ und $R^2$ Wasserstoffatome oder Methylgruppen

$R^3$ ein Wasserstoffatom oder eine Methyl-, Hydroxy- oder $C_{1-6}$-Alkoxygruppe

$R^4$ ein Wasserstoffatom oder eine Methyl- oder Methoxygruppe

$R^5$ ein Wasserstoff- oder Halogenatom oder eine Methoxy- oder $C_{1-4}$-Alkylgruppe

A einen gegebenenfalls mit $C_{1-4}$-Alkylgruppen substituierten Methylen- oder Ethylenrest oder die

Gruppen —CH=CH—, —CHOH—$CH_2$— oder —CO—$CH_2$— und

$R^6$ ein Wasserstoffatom, eine Methyl- oder Nitrilgruppe, den Tetrazolyl- oder 2-Oxazolinylrest, eine $C_{2-10}$-Ketalgruppe oder die Reste —$CHR^7$—$OR^8$, —$CHR^5$—$NR^9R^{10}$, —$COR^{11}$, —$CR^{12}$=CH—$COOR^{13}$ oder —$CR^{12}$=CH—CO—$NR^{14}R^{15}$ bedeuten, worin

$R^7$ ein Wasserstoffatom oder eine $C_{1-4}$-Alkylgruppe

$R^8$ ein Wasserstoffatom, eine $C_{1-4}$-Alkyl-, $C_{1-20}$-Alkanoyl-, eine gegebenenfalls substituierte Benzoylgruppe oder die Reste —P(O) $(OR^{13})_2$ oder —P(O) $(NR^{14}R^{15})_2$ (mit $R^{13}$ in der Bedeutung eines Wasserstoffatoms, einer gegebenenfalls durch eine Hydroxygruppe substituierten $C_{1-8}$-Alkylgruppe, einer gegebenenfalls substituierten Aryl- oder gegebenenfalls im Arylteil substituierten Aralkylgruppe, und mit $R^{14}$ und $R^{15}$ in der Bedeutung von Wasserstoffatomen, gegebenenfalls durch eine Hydroxygruppe substituierten $C_{1-6}$-Alkylgruppen, gegebenenfalls substituierten Aralkyl- oder Arylgruppen, oder $R^{14}$ und $R^{15}$ zusammen mit dem Stickstoffatom, an das sie gebunden sind, in der Bedeutung eines heterocyclischen Restes

$R^9$ und $R^{10}$ Wasserstoffatome, $C_{1-4}$-Alkyl-, $C_{1-6}$-Alkanoyl- oder gegebenenfalls substituierte Benzoylgruppen oder zusammen mit dem Stickstoffatom, an das sie gebunden sind, einen heterocyclischen Rest

$R^{11}$ ein Wasserstoff- oder Halogenatom, eine $C_{1-4}$-Alkylgruppe oder die Reste —$NR^{14}R^{15}$ oder —$OR^{13}$ (mit $R^{13}$, $R^{14}$ und $R^{15}$ in der oben angegebenen Bedeutung) und

$R^{12}$ ein Wasserstoffatom oder eine Methylgruppe darstellen,

sowie gegebenenfalls deren physiologisch verträgliche Salze, dadurch gekennzeichnet, daß man

a) falls $R^6$ eine Cyanogruppe bedeutet — Stilbene der Formel II

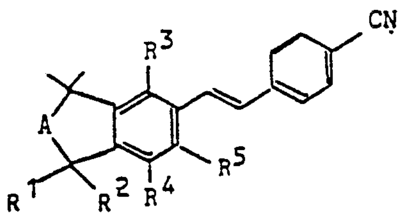

(II)

in der $R^1$-$R^5$ und A die oben angegebene Bedeutung haben, halogeniert und anschließend 2 Mole Halogenwasserstoff abspaltet, oder

b) falls $R^6$ ein Wasserstoffatom, eine Carboxyl-, Nitril- oder Formylgruppe bedeutet — ein α-Chlorbenzylphosphonat der Formel III

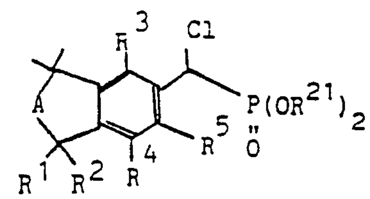

(III)

in der $R^1$-$R^5$ und A die oben angegebene Bedeutung haben, und $R^{21}$ eine $C_{1-3}$-Alkylgruppe bedeutet, mit Aldehyden der Formel IV

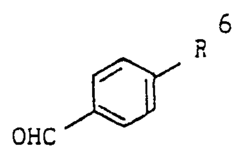

(IV)

umsetzt oder

c) falls $R^6$ eine Methyl- oder Nitrilgruppe oder einen 1,3-Dioxolan-2-yl-rest bedeutet — ein α-Chlorbenzylphosphonat der Formel V,

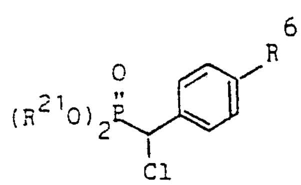

(V)

worin $R^{21}$ die oben angegebene Bedeutung hat, mit Aldehyden der Formel VI

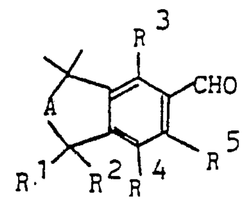

(VI)

worin $R^1$-$R^5$ und A die oben angegebene Bedeutung haben, umsetzt oder

d) falls $R^6$ dasselbe wie unter b) angegeben bedeutet — Monoarylacetylene der Formel VII

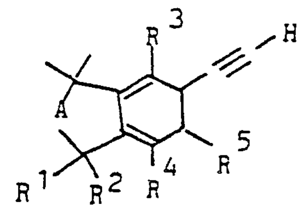

(VII)

worin $R^1$-$R^5$ und A die oben angegebene Bedeutung haben, mit einem Arylhalogenid der Formel VIII

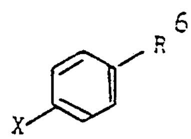

(VIII)

in Gegenwart eines Katalysators, und einer Base umsetzt und die so erhaltenen Verbindungen gegebenenfalls nach Standard-methoden in weitere Verbindungen der Formel I umwandelt.

2. Verfahren gemäß Anspruch 1, dadurch gekennzeichnet, daß man 4-[(5,6,7,8-Tetrahydro-5,5,8,8-tetramethyl-2-naphthyl) ethinyl] benzoesäure herstellt.

3. Verfahren gemäß Anspruch 1, dadurch gekennzeichnet, daß man 4-[(5,6,7,8-Tetrahydro-5,5,8,8-tetramethyl-2-naphthyl) ethinyl] benzonitril herstellt.

4. Verfahren gemäß Anspruch 1, dadurch gekennzeichnet, daß man 4-[(5,6,7,8-Tetrahydro-5,5,8,8-tetramethyl-2-naphthyl) ethinyl] benzoesäureethylester herstellt.

5. Verfahren gemäß Anspruch 1, dadurch gekennzeichnet, daß man 2-(5,6,7,8-Tetrahydro-5,5,8,8-tetramethyl-2-naphthyl)-1[4-(4-(1H-tetrazolyl-5-yl)-phenyl] acetylen herstellt.

6. Verfahren gemäß Anspruch 1, dadurch gekennzeichnet, daß man (5,6,7,8-Tetrahydro-5,5,8,8-tetramethyl-2-naphthyl)-(4-tolyl)-acetylen herstellt.

7. Verfahren gemäß Anspruch 1, dadurch gekennzeichnet, daß man 4-[(5,6,7,8-Tetrahydro-5,5,8,8-tetramethyl-2-naphthyl) ethinyl]-benzaldehyd herstellt.

8. Verfahren gemäß Anspruch 1, dadurch gekennzeichnet, daß man 4-[(5,6,7,8-Tetrahydro-5,5,8,8-tetramethyl-2-naphthyl) ethinyl]-benzylalkohol herstellt.

**Claims** (for the Contracting States : BE, CH, DE, FR, GB, IT, LI, NL, SE)

1. A diarylacetylene of the formula I

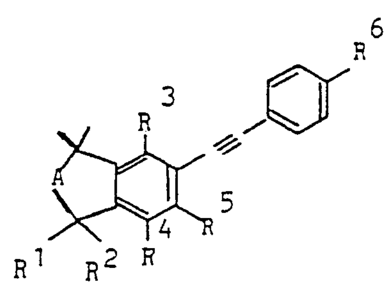

(I)

where

$R^1$ and $R^2$ are each hydrogen or methyl,

$R^3$ is hydrogen, methyl, hydroxyl or $C_1$-$C_6$-alkoxy,

$R^4$ is hydrogen, methyl or methoxy,

$R^5$ is hydrogen, halogen, methoxy or $C_1$-$C_4$-alkyl,

A is a methylene or ethylene radical which is unsubstituted or substituted by $C_1$-$C_4$-alkyl or is —CH=CH—, —CHOH—$CH_2$— or —CO—$CH_2$—, and

$R^6$ is hydrogen, methyl, nitrile, tetrazolyl, 2-oxazolinyl, $C_2$-$C_{10}$-ketal or a radical —$CHR^7$—$OR^8$, —$CHR^7$—$NR^9R^{10}$, —$COR^{11}$, —$CR^{12}$=CH—$COOR^{13}$ or —$CR^{12}$=CH—CO—$NR^{14}R^{15}$, where

$R^7$ is hydrogen or $C_1$-$C_4$-alkyl,

$R^8$ is hydrogen, $C_1$-$C_4$-alkyl, $C_1$-$C_{20}$-alkanoyl, unsubstituted or substituted benzoyl or a radical —$P(O)(OR^{13})_2$ or —$P(O)(NR^{14}R^{15})_2$ (where $R^{13}$ is hydrogen, unsubstituted or hydroxyl-substituted $C_1$-$C_8$-alkyl, unsubstituted or substituted aryl or aralkyl which is unsubstituted or substituted in the aryl moiety, and $R^{14}$ and $R^{15}$ are each hydrogen, unsubstituted or hydroxyl-substituted $C_1$-$C_6$-alkyl, or an unsubstituted or substituted aralkyl or aryl group, or $R^{14}$ and $R^{15}$, together with the nitrogen atom to which they are bonded, form a heterocyclic radical,

$R^9$ and $R^{10}$ are each hydrogen, $C_1$-$C_4$-alkyl, $C_1$-$C_6$-alkanoyl or unsubstituted or substituted benzoyl or $R^9$ and $R^{10}$, together with the nitrogen atom to which they are bonded, form a heterocyclic radical

$R^{11}$ is hydrogen, halogen, $C_1$-$C_4$-alkyl or a radical $NR^{14}R^{15}$ or —$OR^{13}$ (where $R^{13}$, $R^{14}$ and $R^{15}$ have the above meanings) and

$R^{12}$ is hydrogen or methyl, and, where relevant, its physiologically tolerated salts.

2. 4-[(5,6,7,8-tetrahydro-5,5,8,8-tetramethylnaphth-2-yl)-ethynyl]-benzoic acid.
3. 4-[(5,6,7,8-tetrahydro-5,5,8,8-tetramethylnaphth-2-yl)-ethynyl]-benzonitrile.
4. 4-[(5,6,7,8-tetrahydro-5,5,8,8-tetramethylnaphth-2-yl)-ethynyl]-benzoate.
5. 2-(5,6,7,8-tetrahydro-5,5,8,8-tetramethylnaphth-2-yl)-1-[4-(4-(1H-tetrazol-5-yl)-phenyl]-acetylene.
6. (5,6,7,8-tetrahydro-5,5,8,8-tetramethylnaphth-2-yl)-(4-tolyl)-acetylene.
7. 4-[(5,6,7,8-tetrahydro-5,5,8,8-tetramethylnaphth-2-yl)-ethynyl]-benzaldehyde.
8. 4-[(5,6,7,8-tetrahydro-5,5,8,8-tetramethylnaphth-2-yl)-ethynyl]-benzyl alcohol.
9. A process for the preparation of a diarylacetylene of the formula I as claimed in claim 1, wherein

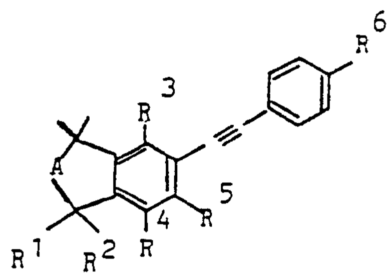

(I)

a) where $R^6$ is cyano, a stilbene of the formula II

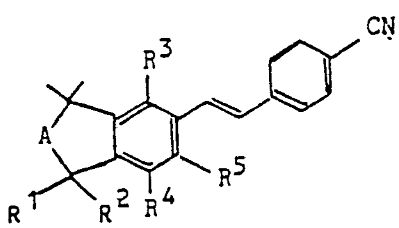

(II)

where $R^1$, $R^2$, $R^3$, $R^4$, $R^5$ and A have the above meanings, is halogenated and 2 moles of hydrogen halide are then eliminated, or

b) where $R^6$ is hydrogen, carboxyl, nitrile or formyl, an α-chlorobenzylphosphonate of the formula III

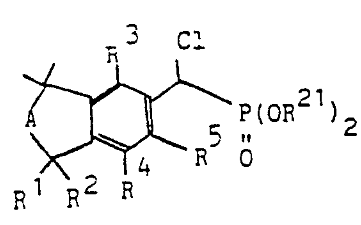

(III)

where $R^1$, $R^2$, $R^3$, $R^4$, $R^5$ and A have the above meanings, and $R^{21}$ is $C_1$-$C_3$-alkyl, is reacted with an aldehyde of the formula IV

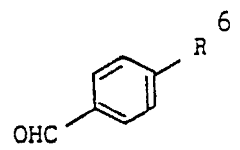

(IV)

or

c) where $R^6$ is methyl, nitrile or 1,3-dioxolan-2-yl, an α-chlorobenzylphosphonate of the formula V

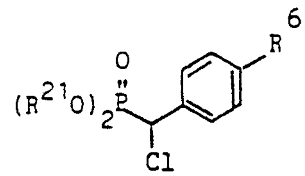

(V)

where $R^{21}$ has the above meanings, is reacted with an aldehyde of the formula VI

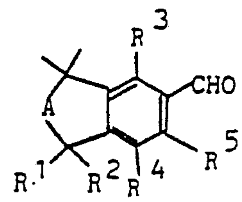

(VI)

where $R^1$, $R^2$, $R^3$, $R^4$, $R^5$ and A have the above meanings, or d) where $R^6$ has the same meanings as stated under b), a monoarylacetylene of the formula VII

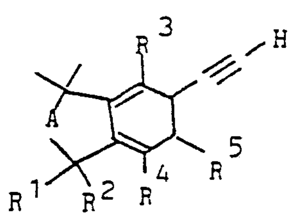

(VII)

where $R^1$, $R^2$, $R^3$, $R^4$, $R^5$ and A have the above meanings, is reacted with an aryl halide of the formula VIII

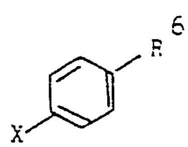

(VIII)

in the presence of a catalyst and of a base, and, if desired, the resulting compound is converted to a further compound of the formula I by a standard method.

10. Use of an arylacetylene of the formula I as claimed in claim 1 for use in controlling diseases.

**Claims** (for the Contracting State AT)

1. A process for the preparation of a diarylacetylene of the formula I

(See Formula page 21)

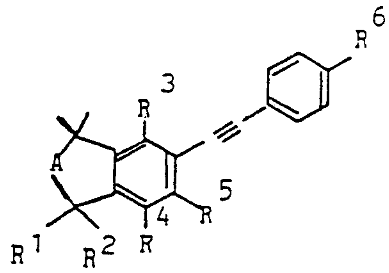

(I)

where

$R^1$ and $R^2$ are each hydrogen or methyl,

$R^3$ is hydrogen, methyl, hydroxyl or $C_1$-$C_6$-alkoxy,

$R^4$ is hydrogen, methyl or methoxy,

$R^5$ is hydrogen, halogen, methoxy or $C_1$-$C_4$-alkyl,

A is a methylene or ethylene radical which is unsubstituted or substituted by $C_1$-$C_4$-alkyl or is —CH=CH—, —CHOH—$CH_2$— or —CO—$CH_2$—, and

$R^6$ is hydrogen, methyl, nitrile, tetrazolyl, 2-oxazolinyl, $C_2$-$C_{10}$-ketal or a radical —$CHR^7$—$OR^8$, —$CHR^7$—$NR^9R^{10}$, —$COR^{11}$, —$CR^{12}$=CH—$COOR^{13}$ or —$CR^{12}$=CH—CO—$NR^{14}R^{15}$, where

$R^7$ is hydrogen or $C_1$-$C_4$-alkyl,

$R^8$ is hydrogen, $C_1$-$C_4$-alkyl, $C_1$-$C_{20}$-alkanoyl, unsubstituted or substituted benzoyl or a radical —$P(O)(OR^{13})_2$ or —$P(O)(NR^{14}R^{15})_2$ (where $R^{13}$ is hydrogen, unsubstituted or hydroxyl-substituted $C_1$-$C_8$-alkyl, unsubstituted or substituted aryl or aralkyl which is unsubstituted or substituted in the aryl moiety, and $R^{14}$ and $R^{15}$ are each hydrogen, unsubstituted or hydroxyl-substituted $C_1$-$C_6$-alkyl, or an unsubstituted or substituted aralkyl or aryl group, or $R^{14}$ and $R^{15}$, together with the nitrogen atom to which they are bonded, form a heterocyclic radical, $R^9$ and $R^{10}$ are each hydrogen, $C_1$-$C_4$-alkyl, $C_1$-$C_6$-alkanoyl or unsubstituted or substituted benzoyl or

$R^9$ and $R^{10}$, together with the nitrogen atom to which they are bonded, form a heterocyclic radical,

$R^{11}$ is hydrogen, halogen, $C_1$-$C_4$-alkyl or a radical $NR^{14}R^{15}$ or —$OR^{13}$ (where $R^{13}$, $R^{14}$ and $R^{15}$ have the above meanings) and

$R^{12}$ is hydrogen or methyl, and, where relevant, its physiologically tolerated salts, wherein

a) where $R^6$ is cyano, a stilbene of the formula II

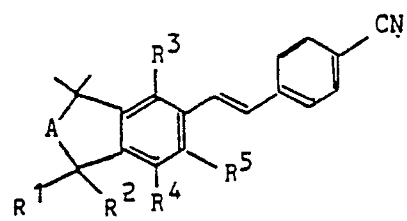

(II)

where $R^1$, $R^2$, $R^3$, $R^4$, $R^5$ and A have the above meanings, is halogenated and 2 moles of hydrogen halide are then eliminated, or

b) where $R^6$ is hydrógen, carboxyl, nitrile or formyl, an α-chlorobenzylphosphonate of the formula III

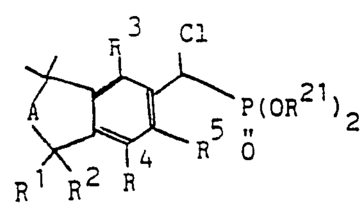

(III)

where $R^1$, $R^2$, $R^3$, $R^4$, $R^5$ and A have the above meanings, and $R^{21}$ is $C_1$-$C_3$-alkyl, is reacted with an aldehyde of the formula IV

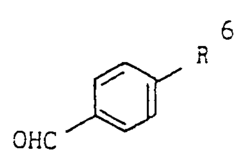

(IV)

or

c) where $R^6$ is methyl, nitrile or 1,3-dioxolan-2-yl, an α-chlorobenzylphosphonate of the formula V

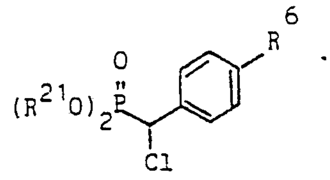

(V)

where $R^{21}$ has the above meanings, is reacted with an aldehyde of the formula VI

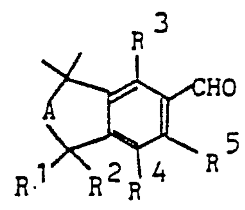

(VI)

where $R^1$, $R^2$, $R^3$, $R^4$, $R^5$ and A have the above meanings, or

d) where $R^6$ has the same meanings as stated under b), a monoarylacetylene of the formula VII

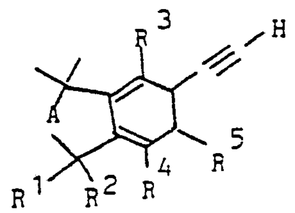

(VII)

where $R^1$, $R^2$, $R^3$, $R^4$, $R^5$ and A have the above meanings, is reacted with an aryl halide of the formula VIII

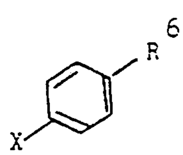

(VIII)

in the presence of a catalyst and of a base, and, if desired, the resulting compound is converted to a further compound of the formula I by a standard method.

2. A process as claimed in claim 1, wherein 4-[(5,6,7,8-tetrahydro-5,5,8,8-tetramethylnaphth-2-yl)-ethynyl]-benzoic acid is prepared.

3. A process as claimed in claim 1, wherein 4-[(5,6,7,8-tetrahydro-5,5,8,8-tetramethylnaphth-2-yl)-ethynyl]-benzonitrile is prepared.

4. A process as claimed in claim 1, wherein 4-[(5,6,7,8-tetrahydro-5,5,8,8-tetramethylnaphth-2-yl)-ethynyl]-benzoate is prepared.

5. A process as claimed in claim 1, wherein 2-(5,6,7,8-tetrahydro-5,5,8,8-tetramethylnaphth-2-yl)-1-[4-(4-(1H-tetrazol-5-yl)-phenyl]-acetylene is prepared.

6. A process as claimed in claim 1, wherein (5,6,7,8-tetrahydro-5,5,8,8-tetramethylnaphth-2-yl)-(4-tolyl)-acetylene is prepared.

7. A process as claimed in claim 1, wherein 4-[(5,6,7,8-tetrahydro-5,5,8,8-tetramethylnaphth-2-yl)-ethynyl]-benzaldehyde is prepared.

8. A process as claimed in claim 1, wherein 4-[(5,6,7,8-tetrahydro-5,5,8,8-tetramethylnaphth-2-yl)-ethynyl]-benzyl alcohol is prepared.

**Revendications** (pour les Etats contractants : BE, CH, DE, FR, GB, IT, LI, NL, SE)

1. Diarylacétylènes de formule I

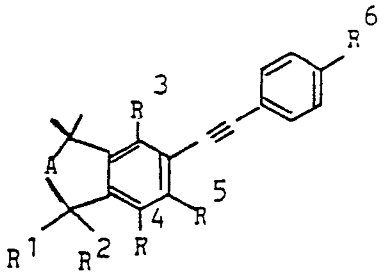

(I)

dans laquelle

$R^1$ et $R^2$ représentent des atomes d'hydrogène ou des groupes méthyle

$R^3$, un atome d'hydrogène ou un groupe méthyle, hydroxy ou alcoxy en $C_1$-$C_6$,

$R^4$, un atome d'hydrogène ou un groupe méthyle ou méthoxy,

$R^5$, un atome d'hydrogène ou d'halogène ou un groupe méthoxy ou alkyle en $C_{1-4}$

A, un reste méthylène ou éthylène éventuellement substitué par des groupes alkyle en $C_{1-4}$, ou les groupes —CH=CH—, —CHOH—$CH_2$— ou —CO—$CH_2$—, et

$R^6$ un atome d'hydrogène, un groupe méthyle ou nitrile, le reste tétrazolyle ou 2-oxazolinyle, un groupe cétal en $C_{2-10}$ ou les restes —$CHR^7$—$OR^8$, —$CHR^7$—$NR^9R^{10}$, —$COR^{11}$, —$CR^{12}$=CH—$COOR^{13}$ ou —$CR^{12}$=CH—CO—$NR^{14}R^{15}$, dans lesquels

$R^7$ représente un atome d'hydrogène ou un groupe alkyle en $C_{1-4}$

$R^8$ un atome d'hydrogène, un groupe alkyle en $C_{1-4}$, alcanoyle en $C_{1-20}$, un groupe benzoyle éventuellement substitué ou les restes —$P(O)(OR^{13})_2$ ou —$P(O)(NR^{14}R^{15})_2$ (avec $R^{13}$ signifiant un atome d'hydrogène, un groupe alkyle en $C_{1-8}$, éventuellement substitué par un groupe hydroxy, un groupe aryle éventuellement substitué ou un groupe aralkyle éventuellement substitué dans la partie aryle, et $R^{14}$ et $R^{15}$ signifiant des atomes d'hydrogène, groupes alkyle en $C_{1-6}$ éventuellement substitués par un groupe hydroxy, des groupes aralkyle ou aryle éventuellement substitués, ou $R^{14}$ et $R^{15}$, ensemble avec l'atome d'azote auquel ils sont liés, signifiant un reste hétérocyclique

$R^9$ et $R^{10}$ représentent des atomes d'hydrogène, des groupes alkyle en $C_{1-4}$, alcanoyle en $C_{1-6}$ ou des groupes benzoyle, éventuellement substitués ou, ensemble avec l'atome d'azote auquel ils sont liés, un reste hétérocyclique,

$R^{11}$, un atome d'hydrogène ou d'halogène, un groupe alkyle en $C_{1-4}$ ou les restes —$NR^{14}R^{15}$ ou —$OR^{13}$ (avec $R^{13}$, $R^{14}$ et $R^{15}$ ayant la signification donnée plus haut) et

$R^{12}$ représente un atome d'hydrogène ou un groupe méthyle

ainsi qu'éventuellement leurs sels tolérables physiologiquement.

2. Acide 4-[(5,6,7,8-tétrahydro-5,5,8,8-tétraméthyl-2-naphtyl) éthinyl] benzoïque.
3. 4-[(5,6,7,8-tétrahydro-5,5,8,8-tétraméthyl-2-naphtyl) éthinyl]-benzonitrile.
4. 4-[(5,6,7,8-tétrahydro-5,5,8,8-tétraméthyl-2-naphtyl) éthynyl] benzoate d'éthyle.
5. 2-(5,6,7,8-tétrahydro-5,5,8,8-tétraméthyl-2-naphtyl)-1[4-(4-(1H-tétrazolyl-5-yl)-phényl] acétylène.
6. (5,6,7,8-tétrahydro-5,5,8,8-tétraméthyl-2-naphtyl)-(4-tolyl)-acétylène.
7. 4-[(5,6,7,8-tétrahydro-5,5,8,8-tétraméthyl-2-naphtyl) éthinyl]-benzaldéhyde.
8. Alcool 4-[(5,6,7,8-tétrahydro-5,5,8,8-tétraméthyl-2-naphtyl) éthinyl]-benzylique.
9. Procédé de préparation de diarylacétylènes de formule I selon la revendication 1,

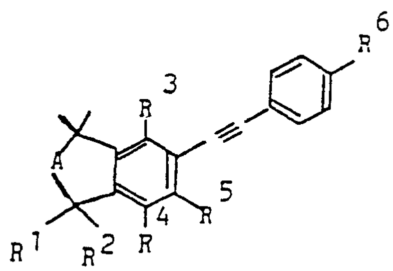

(I)

caractérisé par le fait que

a) dans le cas où $R^6$ représente un groupe cyano-, on halogène des stilbènes de formule II

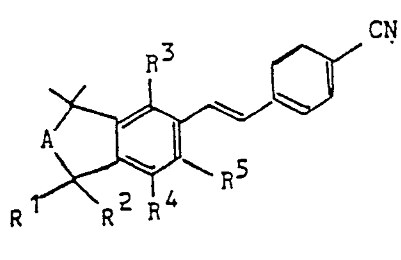

(II)

dans laquelle $R^1$-$R^5$ et A ont les significations susmentionnées, puis on élimine 2 moles d'hydracide ou

b) dans le cas où $R^6$ représente un atome d'hydrogène, un groupe carboxyle, nitrile ou formyle, on fait réagir un α-chlorobenzylphosphonate de formule III

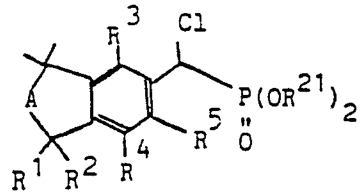

(III)

dans laquelle $R^1$-$R^5$ et A ont la signification sus-indiquée et $R^{21}$ représente un groupe alkyle en $C_{1-3}$, avec des aldéhydes de formule IV

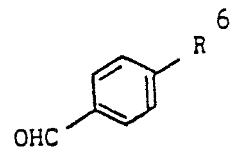

(IV)

ou

c) dans le cas où $R^6$ représente un groupe méthyle ou nitrile ou un reste 1,3-dioxolan-2-yle- on fait réagir un α-chlorobenzylphosphonate de formule V

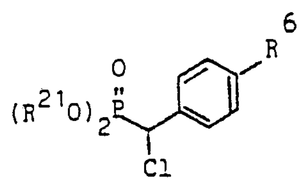

(V)

où $R^{21}$ a la signification sus-indiquée, avec des aldéhydes de formule VI

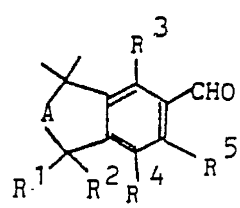

(VI)

où $R^1$-$R^5$ et A ont les significations sus-indiquées, ou

d) dans le cas où $R^6$ représente la même chose que ce qui est indiqué sous b) on fait réagir, en présence d'un catalyseur et d'une base, des monoarylacétylènes de formule VII

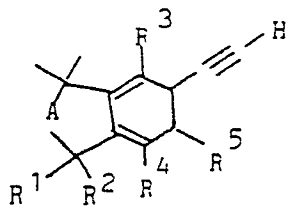

(VII)

où $R^1$-$R^5$ et A ont les significations sus-indiquées, avec un halogénure d'aryle de formule VIII

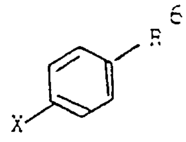

(VIII)

et l'on transforme les composés ainsi obtenus, éventuellement selon des méthodes normalisées, en d'autres composés de formule I.

10. Utilisation des arylacétylènes de formule I selon la revendication 1 pour emploi dans la lutte contre des maladies.

**Revendications** (pour l'Etat contractant AT)

1. Procédé de préparation de diarylacétylènes de formule I

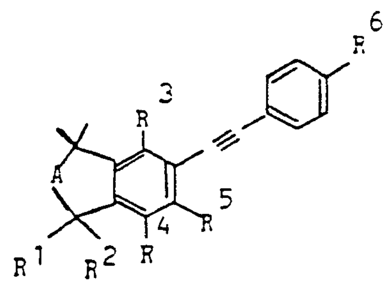

(I)

dans laquelle

$R^1$ et $R^2$ représentent des atomes d'hydrogène ou des groupes méthyle

$R^3$, un atome d'hydrogène ou un groupe méthyle, hydroxy ou alcoxy en $C_1$-$C_6$,

$R^4$, un atome d'hydrogène ou un groupe méthyle ou méthoxy,

$R^5$, un atome d'hydrogène ou d'halogène ou un groupe méthoxy ou alkyle en $C_{1-4}$

A, un reste méthylène ou éthylène éventuellement substitué par des groupes alkyle en $C_{1-4}$, ou les groupes —CH=CH—, —CHOH—$CH_2$— ou —CO—$CH_2$—, et

$R^6$ un atome d'hydrogène, un groupe méthyle ou nitrile, le reste tétrazolyle ou 2-oxazolinyle, un groupe cétal en $C_{2-10}$ ou les restes —$CHR^7$—$OR^8$, —$CHR^7$—$NR^9R^{10}$, —$COR^{11}$, —$CR^{12}$=CH—$COOR^{13}$ ou —$CR^{12}$=CH—CO—$NR^{14}R^{15}$, dans lesquels

$R^7$ représente un atome d'hydrogène ou un groupe alkyle en $C_{1-4}$

$R^8$ un atome d'hydrogène, un groupe alkyle en $C_{1-4}$, alcanoyle en $C_{1-20}$, un groupe benzoyle éventuellement substitué ou les restes —$P(O)(OR^{13})_2$ ou —$P(O)(NR^{14}R^{15})_2$ (avec $R^{13}$ signifiant un atome d'hydrogène, un groupe alkyle en $C_{1-8}$, éventuellement substitué par un groupe hydroxy, un groupe aryle éventuellement substitué ou un groupe aralkyle éventuellement substitué dans la partie aryle, et $R^{14}$ et $R^{15}$ signifiant des atomes d'hydrogène, groupes alkyle en $C_{1-6}$ éventuellement substitués par un groupe hydroxy, des groupes aralkyle ou aryle éventuellement substitués, ou $R^{14}$ et $R^{15}$, ensemble avec l'atome d'azote auquel ils sont liés, signifiant un reste hétérocyclique

$R^9$ et $R^{10}$ représentent des atomes d'hydrogène, des groupes alkyle en $C_{1-4}$, alcanoyle en $C_{1-6}$ ou des groupes benzoyle, éventuellement substitués ou, ensemble avec l'atome d'azote auquel ils sont liés, un reste hétérocyclique,

$R^{11}$, un atome d'hydrogène ou d'halogène, un groupe alkyle en $C_{1-4}$ ou les restes —$NR^{14}R^{15}$ ou —$OR^{13}$ (avec $R^{13}$, $R^{14}$ et $R^{15}$ ayant la signification donnée plus haut) et

$R^{12}$ représente un atome d'hydrogène ou un groupe méthyle

ainsi qu'éventuellement leurs sels tolérables physiologiquement.

caractérisé par le fait que

a) dans le cas où $R^6$ représente un groupe cyano-, on halogène des stilbènes de formule II

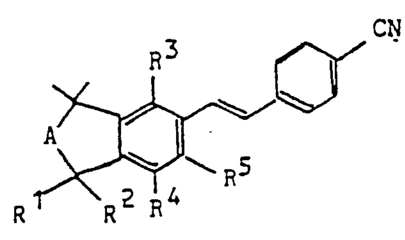

(II)

dans laquelle $R^1$-$R^5$ et A ont les significations susmentionnées, puis on élimine 2 moles d'hydracide ou

b) dans le cas où $R^6$ représente un atome d'hydrogène, un groupe carboxyle, nitrile ou formyle, on fait réagir un α-chlorobenzylphosphonate de formule III

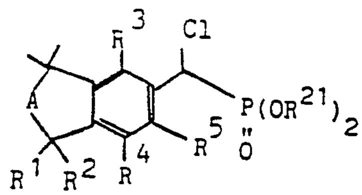

(III)

dans laquelle $R^1$-$R^5$ et A ont la signification sus-indiquée et $R^{21}$ représente un groupe alkyle en $C_{1-3}$, avec des aldéhydes de formule IV

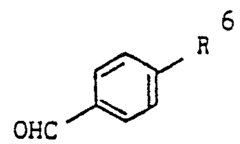

(IV)

ou

c) dans le cas où $R^6$ représente un groupe méthyle ou nitrile ou un reste 1,3-dioxolan-2-yle- on fait réagir un α-chlorobenzylphosphonate de formule V

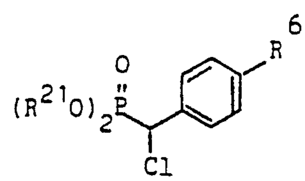

(V)

où $R^{21}$ a la signification sus-indiquée, avec des aldéhydes de formule VI

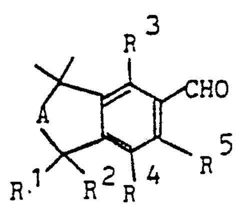

(VI)

où $R^1$-$R^5$ et A ont les significations sus-indiquées, ou

d) dans le cas où $R^6$ représente la même chose que ce qui est indiqué sous b) on fait réagir, en présence d'un catalyseur et d'une base, des monoarylacétylènes de formule VII

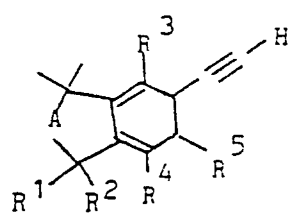

(VII)

où $R^1$-$R^5$ et A ont les significations sus-indiquées, avec un halogénure d'aryle de formule VIII

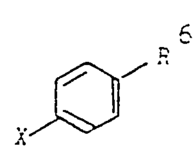

(VIII)

et l'on transforme les composés ainsi obtenus, éventuellement selon des méthodes normalisées, en d'autres composés de formule I.

2. Procédé selon la revendication 1, caractérisé par le fait que l'on prépare de l'acide 4-[(5,6,7,8-tétrahydro-5,5,8,8-tétramétyl-2-naphtyl) éthinyl] benzoïque.

3. Procédé selon la revendication 1, caractérisé par le fait que l'on prépare du 4-[(5,6,7,8-tétrahydro-5,5,8,8-tétraméthyl-2-naphtyl) éthinyl]-benzonitrile.

4. Procédé selon la revendication 1, caractérisé par le fait que l'on prépare du 4-[(5,6,7,8-tétrahydro-5,5,8,8-tétraméthyl-2-naphtyl) éthynyl] benzoate d'éthyle.

5. Procédé selon la revendication 1, caractérisé par le fait que l'on prépare du 2-(5,6,7,8-tétrahydro-5,5,8,8-tétraméthyl-2-naphtyl)-1[4-(4-(1H-tétrazolyl-5-yl)-phényl] acétylène.

6. Procédé selon la revendication 1, caractérisé par le fait que l'on prépare du (5,6,7,8-tétrahydro-5,5,8,8-tétraméthyl-2-naphtyl)-(4-tolyl)-acétylène.

7. Procédé selon la revendication 1, caractérisé par le fait que l'on prépare du 4-[(5,6,7,8-tétrahydro-5,5,8,8-tétraméthyl-2-naphtyl) éthinyl]-benzaldéhyde.

8. Procédé selon la revendication 1, caractérisé par le fait que l'on prépare de l'Alcool 4-[(5,6,7,8-tétrahydro-5,5,8,8-tétraméthyl-2-naphtyl) éthinyl]-benzylique.